# EUROPEAN PATENT SPECIFICATION

(11) **EP 3 922 266 B1**
(45) Date of publication and mention of the grant of the patent: **08.04.2026**
(21) Application number: 20753017.1
(22) Date of filing: 07.02.2020
(51) Int. Cl.: A61K 47/54, A61K 47/69, A61P 35/00, A61K 39/39, A61K 45/06, A61K 39/00, A61K 9/127, A61K 39/395, C07K 16/28

(54) **TOLL-LIKE RECEPTOR 7 OR 8 AGONIST-CHOLESTEROL COMPLEX, AND USE OF SAME**
TOLL-LIKE-REZEPTOR-7- ODER -8-AGONIST-CHOLESTERIN-KOMPLEX UND VERWENDUNG DAVON
COMPLEXE AGONISTE DU RÉCEPTEUR DE TYPE TOLL 7 OU 8-CHOLESTÉROL, ET SON UTILISATION

(30) Priority: 08.02.2019 KR 20190014756
(43) Date of publication of application: 15.12.2021
(73) Proprietor: PROGENEER, INC., Guro-gu Seoul 08380 (KR)
(72) Inventor: LIM, Yong Taik, Seongnam-si, Gyeonggi-do 13599 (KR); SHIN, Hong Sik, Anyang-si, Gyeonggi-do 14043 (KR); REN, Long, Suwon-si, Gyeonggi-do 16418 (KR)
(74) Representative: Dehns
(86) International application number: PCT/KR2020/001753
(87) International publication number: WO 2020/162705

(56) References cited:
- EP-A1- 2 674 170
- WO-A1-2017/102654
- US-A1- 2013 336 996
- US-A1- 2018 273 560
- US-A1- 2018 362 560
- DIEBOLD SANDRA S ET AL: "Nucleic acid agonists for Toll-like receptor 7 are defined by the presence of uridine ribonucleotides", EUROPEAN JOURNAL OF IMMUNOLOGY, WILEY-VCH, HOBOKEN, USA, vol. 36, no. 12, 1 December 2006 (2006-12-01), pages 3256 - 3267, XP008101287, ISSN: 0014-2980, DOI: 10.1002/EJI.200636617
- BOB J. IGNACIO ET AL: "Toll-like Receptor Agonist Conjugation: A Chemical Perspective", BIOCONJUGATE CHEMISTRY, vol. 29, no. 3, 29 January 2018 (2018-01-29), US, pages 587 - 603, XP055598375, ISSN: 1043-1802, DOI: 10.1021/acs.bioconjchem.7b00808
- JIANG ZHU ET AL: "Local administration of a novel Toll-like receptor 7 agonist in combination with doxorubicin induces durable tumouricidal effects in a murine model of T cell lymphoma", JOURNAL OF HEMATOLOGY & ONCOLOGY, BIOMED CENTRAL LTD, LONDON UK, vol. 8, no. 1, 4 March 2015 (2015-03-04), pages 21, XP021218354, ISSN: 1756-8722, DOI: 10.1186/S13045-015-0121-9
- IGNACIO, BOB J. ET AL.: "Toll-like receptor agonist conjugation: a chemical perspective", BIOCONJUGATE CHEMISTRY, vol. 29, 2018, pages 587 - 603, XP055598375, DOI: 10.1021/acs.bioconjchem.7b00808
- WILKINSON, ALEXANDER ET AL.: "Lipid conjugation of TLR7 agonist Resiquimod ensures co-delivery with the liposomal Cationic Adjuvant Formulation01(CAF01) but does not enhance immunopotentiation compared to non-conjugated Resiquimod+CAF01", JOURNAL OF CONTROLLED RELEASE, vol. 291, 2018, pages 1 - 10, XP085533440, DOI: 10.1016/j.jconrel.2018.10.002

## Description

### [Technical Field]

The present invention relates to a toll-like receptor 7/8 agonist-cholesterol complex, and more particularly, to a complex in which cholesterol is linked with an active site of a toll-like receptor 7/8 agonist by means of a chemical bond with a cleavable site, and a use thereof.

### [Background Art]

An immune response is a series of responses of activated immune cells against exogenous and endogenous materials, that is, antigens, and when microorganisms such as bacteria or viruses, or foreign bio-substances are introduced into the body, they are recognized by immune cells, which are then activated to secrete a factor such as a cytokine, thereby causing an inflammatory response. Recently, active research on mechanisms in an innate immune response stage that non-specifically acts at the early stage of infection is actively progressing, and among these mechanisms, a toll-like receptor (TLR), which is a gene capable of recognizing a pathogen in the early stage of inflammation, is known to recognize a cell membrane component of a pathogen or a nucleic acid component to induce an immune response, and by using the TLR, studies on various toll-like receptor ligands for activating immune cells are actively progressing.

Among these ligands, a toll-like receptor 7/8 agonist-based material is used as an adjuvant inducing a cellular immune response, and may be imiquimod, resiquimod, dactolisib, gardiquimod, sumanirole, motolimod, vesatolimod, loxoribine, SM360320, CL264, 3M-003, IMDQ, or Compound 54 (US 2012-0294885 A1). The toll-like receptor 7/8 agonist is a toll-like receptor 7/8 agonist in an endosome and known to effectively induce not only humoral immunity but also cellular immunity. However, the toll-like receptor 7/8 agonist has difficulty in being dispersed in an aqueous solution due to its molecular structure. In addition, since such agonist is dissolved only in a special organic solvent such as DMSO or methanol, but is not dissolved in a generally used organic solvent, there is a limitation in preparing various forms of immune activation materials. Therefore, the agonist is commercially used in a cream-type formulation (e.g., Aldara^{®} cream) in which various surfactants are mixed. In some studies, to overcome such a problem, while being prepared in the form of a salt to be dissolved in an aqueous solution, the toll-like receptor 7/8 agonist prepared in a salt form is absorbed into a blood vessel in the body to induce a systemic immune response in the blood vessel, thereby causing many side effects (e.g., cytokine storm, and various non-specific hypersensitivity immune responses). For this reason, the salt-form toll-like receptor 7/8 agonist described above is difficult to use. Due to these side effects, in order to actually use it for treatment, it is necessary to be treated at a lower concentration than an effective dose, and thus may become the cause of reduced efficiency. In some pharmaceutical companies, to overcome such problems, an attempt to prevent direct absorption into a blood vessel by introducing a lipid showing a lipophilic property or directly chemically binding to a polymer chain with a huge size is also being made. However, since the active site of a toll-like receptor 7/8 agonist prepared by the above-mentioned is still exposed to the outside, it still has a potential to cause toxicity by inducing a non-specific immune response in the body.

Accordingly, if a toll-like receptor 7/8 agonist, which is not absorbed into a blood vessel in the body and can be prepared in various forms in which a non-specific immune response is suppressed, is developed, it is expected be widely used as various immune activating agents with low side effects.

EP 2674170 A1 **discloses immunogenic molecule compositions and methods for the modulation of toll-like receptor TLR7 and/or TLR8, and cytosolic nucleic acid sensors, and more particularly to agonists of TLR7 and/or TLR8 and cationic lipids for introducing nucleic acids into cells.**

### [Disclosure]

### [Technical Problem]

To solve the conventional technical problems, the present invention is directed to providing a toll-like receptor 7/8 agonist-cholesterol complex which is designed to temporarily inhibit an immune activation function and recover immune activity in a tumor microenvironment or target cells, and a use thereof.

The present invention relates to technology in which a toll-like receptor 7/8 agonist can be applied to various pharmaceutical formulations to be effectively used in actual clinical practice, and minimize the induction of a non-specific immune response and a cytokine storm in the body.

The present invention relates to the design of the structure of a toll-like receptor 7/8 agonist with kinetically controlled activity, which has an inhibited immune activation function, and then recovers activity in a tumor microenvironment and target immune cells, thereby showing immune activation efficacy.

In addition, the present invention relates to a nanoliposome, nanoemulsion, nanomicelle and a polymer nanoparticle, which include a toll-like receptor 7/8 agonist-cholesterol complex.

In addition, the present invention relates to an adjuvant composition, which includes a cholesterol-toll-like receptor 7/8 agonist complex.

In addition, the present invention relates to a vaccine composition, which includes an adjuvant composition including a cholesterol-toll-like receptor 7/8 agonist complex and an antigen.

In addition, the present invention relates to an immune cell activating composition, which includes a cholesterol-toll-like receptor 7/8 agonist complex.

In addition, the present invention relates to an immunosuppressive cell function-controlling composition, which includes a cholesterol-toll-like receptor 7/8 agonist complex.

In addition, the present invention relates to a cancer immunotherapeutic composition, which includes a cholesterol-toll-like receptor 7/8 agonist complex.

In addition, the present invention relates to an anticancer composition, which includes a cholesterol-toll-like receptor 7/8 agonist complex; and further includes an anticancer agent and an immune checkpoint inhibitor.

However, technical problems to be solved in the present invention are not limited to the above-described problems, and other problems which are not described herein will be fully understood by those of ordinary skill in the art from the following descriptions.

### [Technical Solution]

The present invention provides a toll-like receptor 7/8 agonist-cholesterol complex, in which cholesterol is linked to an amine group (NH₂) of an active site of a toll-like receptor 7/8 agonist.

The bond is a separable form and is a cleavable chemical bond selected from a carbamate, a disulfide, an ester, and/or a peptide. In addition, the active site of the toll-like receptor 7/8 agonist is exposed by cleavage of the bond, thereby kinetically recovering a function within 4 days. The toll-like receptor 7/8 agonist includes at least one selected from the group consisting of an imidazoquinoline-based agonist, a hydroxyadenine-based agonist, a pteridone-based agonist, an aminopyrimidine-based agonist, a benzoazepine-based agonist, and a thia-oxoguanosine-based agonist.

In another embodiment of the present invention, the toll-like receptor 7/8 agonist is preferably an imidazoquinoline-based compound, a 8-hydroxyadenine-based compound, a pteridone-based compound, an 2-aminopyrimidine-based compound, a benzoazepine-based compound, a 7-thia-8-oxoguanosine-based compound, or a combination thereof, and more preferably, imiquimod, resiquimod, dactolisib, gardiquimod, sumanirole, motolimod, vesatolimod, loxoribine, SM360320, CL264, 3M-003, IMDQ, or Compound 54. But any toll-like receptor 7/8 agonist that can exhibit an inactive form by chemically binding of cholesterol to an active site is used without limitation.

In addition, the present invention provides a nanoparticle composition which includes the complex.

In one embodiment of the present invention, the nanoparticles increase immune cell activation efficacy, and preferably, are nanoliposomes, nanomicelles, solid nanoparticles, a nanoemulsion, or polymer nanoparticles, which are including the complex. The "including" may mean a form contained within regardless of a chemical bond, a form of being attached to the surface of nanoparticle, or a form of being sandwiched between a nanoparticle structure, but any form including the complex of the present invention is used without limitation.

In addition, the present invention provides an adjuvant composition including the complex.

In addition, the present invention provides a vaccine composition, which includes the adjuvant composition and an antigen.

In one embodiment of the present invention, the antigen is preferably a protein, a recombinant protein, a glycoprotein, a gene, a peptide, a polysaccharide, a lipopolysaccharide, a polynucleotide, cells, a cell lysate, bacteria, virus or a generally known antigen.

In addition, the present invention provides a composition for controlling an immune function, which includes the complex as an active ingredient.

In one embodiment of the present invention, the composition for controlling an immune function may induce the activation of immune cells in a tumor microenvironment, or control the function of immunosuppressive cells, and preferably, induces immune activation of antigen-presenting cells (e.g. dendritic cells and macrophages), natural killer cells (NK cells) or T cells, or regulates the function of immune cells exhibiting an immunosuppressive action (regulatory T cells (Tregs), myeloid-derived suppressor cells (MDSCs), and M2 macrophages), thereby regulating the immune function in the body.

In addition, the present invention provides a pharmaceutical composition for preventing or treating cancer, which includes the complex as an active ingredient.

In one embodiment of the present invention, the pharmaceutical composition may further include materials that are generally used in cancer treatment, and preferably, further includes a chemotherapeutic agent and an immune checkpoint inhibitor. Since the pharmaceutical composition may include the complex of the present invention to effectively activate the immune function in the body, it may enhance the efficacy of a conventional chemotherapeutic agent or immune checkpoint inhibitor.

In another embodiment of the present invention, the pharmaceutical composition inhibits cancer proliferation, metastasis and recurrence, or resistance to an anti-cancer therapy, but as long as it is part of a generally used method for treating cancer, it may be used without limitation.

In still another embodiment of the present invention, the cancer is preferably breast cancer, colorectal cancer, rectal cancer, lung cancer, colon cancer, thyroid cancer, oral cancer, pharyngeal cancer, laryngeal cancer, cervical cancer, brain cancer, ovarian cancer, bladder cancer, kidney cancer, liver cancer, pancreatic cancer, prostate cancer, skin cancer, tongue cancer, uterine cancer, stomach cancer, bone cancer or blood cancer.

In addition, described herein is a method of preventing or treating cancer, which includes administering the composition including the complex as an active ingredient into a subject.

In addition, the present invention provides the composition including the complex as an active ingredient for use in preventing or treating cancer.

In addition, the present invention provides the composition including the complex as an active ingredient for use in preventing or treating an infectious disease.

In addition, the present invention provides the complex for producing a drug for use in in preventing or treating cancer.

In addition, described herein is a method of preparing a toll-like receptor 7/8 agonist-cholesterol complex, which includes chemically bonding cholesterol to an amine group (NH₂) of an active site of a toll-like receptor 7/8 agonist using a cleavable linker which is a carbamate, a disulfide, an ester, a peptide, or a combination thereof.

In one embodiment of the present invention, the preparation method may include mixing and reacting the toll-like receptor 7/8 agonist, cholesteryl chloroformate and pyridine in dichloromethane.

In another embodiment of the present invention, the preparation method may include (a) preparing a disulfide cross-linker by dissolving 2-hydroxyethyl disulfide in tetrahydrofuran, adding and dissolving it in a toluene solution, and adding and dissolving N-hydrosuccinimide and triethylamine; (b) preparing a disulfide-cholesterol cross-linker by mixing and reacting the disulfide cross-linker and cholesterol; and (c) mixing and reacting the disulfide-cholesterol cross-linker and a toll-like receptor 7/8 agonist.

In still another embodiment of the present invention, in the mixing in step (b), the disulfide cross-linker and the cholesterol may be mixed in a weight ratio of 3 : 1 to 1 : 1, and during the mixing in step (c), the disulfide-cholesterol cross-linker and the toll-like receptor 7/8 agonist may be mixed in a weight ratio of 4 : 1 to 1 : 1.

### [Advantageous Effects]

A toll-like receptor 7/8 agonist to which cholesterol is chemically linked according to the present invention (cholesterol-toll-like receptor 7/8 agonist complex) cannot only prevent penetration into blood due to increased lipophilicity, but also remarkably reduce side effects and cytotoxicity of a conventional toll-like receptor 7/8 agonist since its immune activation function is inhibited in an environment other than a tumor microenvironment or endosomes in immune cells.

In addition, after delivery to the tumor microenvironment and immune cells, the cholesterol and the toll-like receptor 7/8 agonist are slowly separated and consistently react with the toll-like receptor for a long time by kinetic immune modulation of the active site of the toll-like receptor 7/8 agonist, so that it can remarkably increase a therapeutic effect by increasing the persistence of immune activation of immune cells compared to when the toll-like receptor agonist is used alone.

In addition, since the cholesterol-toll-like receptor 7/8 agonist complex can induce immune activation of antigen-presenting cells (e.g. dendritic cells and macrophages), natural killer cells (NK cells) or T cells, and regulate the function of immune cells exhibiting an immunosuppressive action (regulatory T cells (Tregs), myeloid derived suppressor cells (MDSCs) or M2 macrophages) in a tumor microenvironment, it cannot only exhibit an anticancer effect alone, but also remarkably increase an anticancer effect due to a synergistic effect by co-administration with an immune checkpoint inhibitor or a chemotherapeutic agent.

Moreover, since the cholesterol-toll-like receptor 7/8 agonist complex is based on cholesterol, which is a basic component of a biomembrane, it can be easily prepared in various forms such as a nanoemulsion, a nanoliposome and a nanomicelle in combination with various lipids, and therefore, intracellular delivery efficacy can increase. For this reason, in the present invention, the cholesterol-toll-like receptor 7/8 agonist complex can be prepared in various forms, and is also expected to remarkably increase the therapeutic effect on various diseases by inducing immune activation by being included in various pharmaceutical compositions.

### [Description of Drawings]

FIG. 1 is a diagram illustrating the structure and activation/inactivation mechanism of a toll-like receptor 7/8 agonist-cholesterol complex.
FIG. 2 shows the result of verifying the structure of a toll-like receptor 7/8 agonist according to one embodiment of the present invention through ¹H-NMR.
FIG. 3 shows the result of verifying the structure of a cholesterol-linked toll-like receptor 7/8 agonist according to one embodiment of the present invention through ¹H-NMR.
FIG. 4 shows the result of verifying the structure of a toll-like receptor 7/8 agonist according to one embodiment of the present invention through ¹⁵N-HSQC.
FIG. 5 shows the result of verifying the structure of a cholesterol-linked toll-like receptor 7/8 agonist according to one embodiment of the present invention through ¹⁵N-HSQC.
FIG. 6 shows the enlarged result of verifying the structure of a cholesterol-linked toll-like receptor 7/8 agonist according to one embodiment of the present invention through ¹⁵N-HSQC.
FIG. 7 shows the mechanism of separating a cholesterol-linked toll-like receptor 7/8 agonist according to one embodiment of the present invention into cholesterol and a toll-like receptor 7/8 agonist according to a physiological environment in cells.
FIG. 8 shows the mechanism of separating a cholesterol-disulfide crosslinked toll-like receptor 7/8 agonist according to one embodiment of the present invention from cholesterol due to a physiological environment in cells.
FIG. 9 is a set of schematic diagrams illustrating the shapes of a nanoparticle including a cholesterol-toll-like receptor 7/8 agonist complex.
FIG. 10 is a graph showing the result of confirming that a toll-like receptor 7/8 agonist-cholesterol complex according to one embodiment of the present invention is kinetically separated over time at a constant pH.
FIG. 11 is a graph showing the result of confirming that a toll-like receptor 7/8 agonist-cholesterol complex according to one embodiment of the present invention is kinetically separated over time due to an enzyme.
FIG. 12 is a graph showing the result of confirming the cytotoxicity of a nanoliposome including a toll-like receptor 7/8 agonist-cholesterol complex according to one embodiment of the present invention.
FIG. 13 is a graph showing the result of confirming an immune cell activation indicator (IL-6) of a nanoliposome including a toll-like receptor 7/8 agonist-cholesterol complex according to one embodiment of the present invention.
FIG. 14 is a graph showing the result of confirming an immune cell activation indicator (TNF-alpha) of a nanoliposome including a toll-like receptor 7/8 agonist-cholesterol complex according to one embodiment of the present invention.
FIG. 15 shows the difference in activation of immune cells by a Chol-R848 material synthesized to allow cleavage at a binding site of a toll-like receptor 7/8 agonist-cholesterol complex according to one embodiment of the present invention and a C18-R848 material synthesized to prevent cleavage thereof.
FIG. 16 shows the result of confirming an amount reaching a lymph node and a retention time after a nanoliposome including resquimod and a toll-like receptor 7 or 8 agonist-cholesterol complex according to one embodiment of the present invention are injected into the body.
FIG. 17 shows the result of confirming the immune cell activation efficacy and toxicity of a nanoliposome including a toll-like receptor 7/8 agonist-cholesterol complex according to one embodiment of the present invention.
FIG. 18 shows the result of confirming the tumor growth inhibitory effect and survival rate of a nanoliposome including a toll-like receptor 7/8 agonist-cholesterol complex according to one embodiment of the present invention.
FIG. 19 shows the result of confirming the ability of regulating immune cell activity of a nanoliposome including a toll-like receptor 7/8 agonist-cholesterol complex according to one embodiment of the present invention at a tumor site.
FIG. 20 shows the result of confirming the ability of regulating immune cell activity of a nanoliposome including a toll-like receptor 7/8 agonist-cholesterol complex according to one embodiment of the present invention in the spleen.
FIG. 21 shows the result of confirming the effect of co-administering a nanoliposome including a toll-like receptor 7/8 agonist-cholesterol complex according to one embodiment of the present invention and an immune checkpoint inhibitor in a B 16-OVA animal model.
FIG. 22 shows the result of confirming the effect of co-administering a nanoliposome including a toll-like receptor 7/8 agonist-cholesterol complex according to one embodiment of the present invention and an immune checkpoint inhibitor in a 4T1 animal model.
FIG. 23 shows the result of confirming the effect of co-administering a nanoliposome including a toll-like receptor 7/8 agonist-cholesterol complex according to one embodiment of the present invention and an immune checkpoint inhibitor in a TC1 animal model.
FIG. 24 shows the result of confirming the effect of co-administration with a nanoliposome including a toll-like receptor 7/8 agonist-cholesterol complex according to one embodiment of the present invention and a chemotherapeutic agent.

### [Best Modes of the Invention]

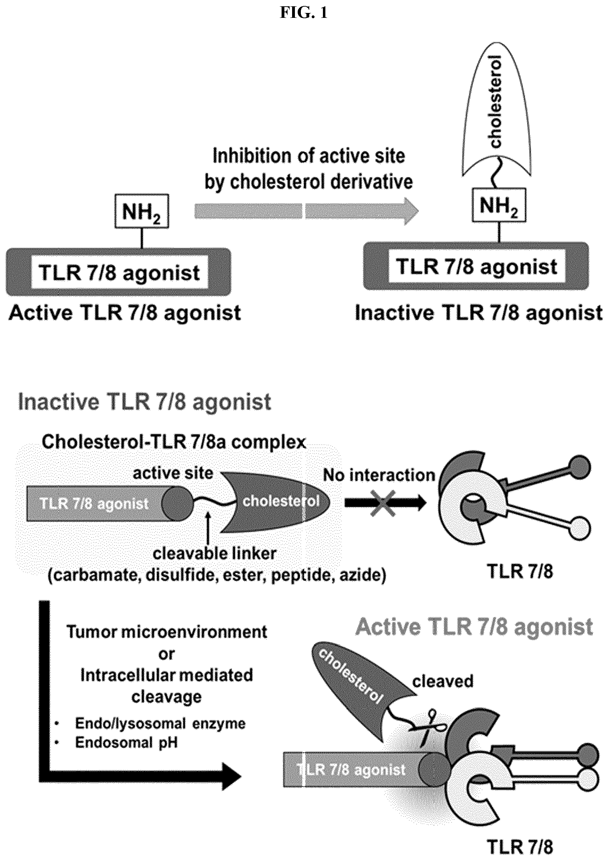

In the present invention, in a toll-like receptor 7/8 agonist-cholesterol complex, a cholesterol group is linked to an amine group (NH₂) of an active site of a toll-like receptor 7/8 agonist in a cleavable form, and thus an immune activation function is temporarily inhibited (FIG. 1). The inhibition may mean that a function of the active site of the toll-like receptor 7/8 agonist is retarded.

In the present invention, the complex is crosslinked to a site at which cleavage is induced due to a tumor microenvironment and/or intracellular environment, particularly, physiological environments (e.g. low pH, enzyme, and glutathione) of an endosome and a lysosome (FIG. 1). Specifically, under a tumor microenvironment, there may be a linkage form that can be regulated so that cleavage occurs by a specific stimulus such as a pH, a temperature, redox potential, ultrasound, an enzyme, a magnetic field or near-infrared light. The linkage is formed by a carbamate, disulfide, ester, or peptide bond.

In addition, in the present invention, in the complex, cholesterol and a toll-like receptor 7/8 agonist may be separated due to various enzymes present in a tumor microenvironment and cells, such as acid phosphatase, acid phyrophosphatase, phosphodiesterase, phosphoprotein phosphatase, phosphatidic acid phosphatase, aryl sulfatase, proteases, cathepsins, collagenase, arylamidase, peptidase, acid ribonuclease, acid deoxyribonuclease, lipases, triglyceride lipase, phospholipase, esterase, carboxyesterase, clucocerebrosidase, galactocerebrosidase, sphingomyelinase, glycosidases, alpha-glucosidase, beta-glucosidase, beta-galactosidase, alpha-mannosidase, alpha-ucosidase, beta-xylosidase, alpha-N-acetylhexosaminidase, beta-N-acetylhexosaminidase, sialidase, lysozyme, hyaluronidase, and beta-glucuronidase.

In the present invention, as the toll-like receptor 7/8 agonist is prepared by chemically linking cholesterol such that it is not absorbed into blood vessels in the body, the disadvantages of a salt-form toll-like receptor 7/8 agonist are overcome.

In the present invention, the complex is easily prepared in various forms such as a nanoliposome, a nanomicelle, and a nanoemulsion by easily interaction with various materials such as various lipid materials and saponins, thereby increasing delivery efficiency into immune cells.

The "cholesterol" used herein is a type of lipid, and encompasses steroid-based organic materials having a hydrophobic property, and the cholesterol may include various derivatives based on a cholesterol structure, and compounds that can be obtained by chemically changing a part of cholesterol. Preferably, the cholesterol includes bile acids (cholic acid, deoxycholic acid, lithocholic acid, and chenodeoxycholic acid), vitamin D, and steroid hormones (testosterone, estradiol, cortisol, aldosterone, prednisolone, and prednisone). In addition, the cholesterol is a material that assists the toll-like receptor 7/8 agonist to be located on the surface and in various forms of nanoparticles, and may be replaced with lipid materials having a similar function thereto, for example, natural lipids such as a phospholipid, and synthetic lipids.

The "toll-like receptor 7/8 agonist-based material" used herein may be selected from the group consisting of toll-like receptor 7 or 8 agonists, such as an imidazoquinoline-based compound, an 8-hydroxyadenine-based compound, a pteridone-based compound, a 2-aminopyrimidine-based compound, a benzoazepine-based compound, and a 7-thia-8-oxoguanosine-based compound. Here, the imidazoquinoline-based compound may include compounds disclosed in WO 2018 196823, WO 2011 049677, WO 2011 027022, WO 2017 102652, and WO 2019 040491, or pharmaceutically acceptable salts thereof. In addition, the 8-hydroxyadenine-based compound may include compounds disclosed in WO 2012 080730, WO 2013 068438, WO 2019 036023, WO 2019 035969, WO 2019 035970, WO 2019 035971, WO 2019 035968, CN 108948016, US 2014 8846697, WO 2016 023511, WO 2017 133683, WO 2017 133686, WO 2017 133684, WO 2017 133687, WO 2017 076346, WO 2018 210298, WO 2018 095426, WO 2018 068593, WO 2018 078149, WO 2018 041763, or pharmaceutically acceptable salts thereof. The pteridone-based compound includes compounds disclosed in US 2010 0143301, WO 2016 007765, WO 2016 044182, WO 2017 035230, WO 2017 219931, WO 2011 057148, and CN 1087 94486, or pharmaceutically acceptable salts thereof. The 2-aminopyrimidine-based compound may include compounds disclosed in WO 2010 133885, WO 2012066335, WO 2012 066336, WO 2012 067268, WO 2013 172479, WO 2012 136834, WO 2014 053516, WO 2014 053595, US 2018 0215720, WO 2012 156498, WO 2014 076221, WO 2016 141092, WO 2018 045144, WO 2015 014815, WO 2018 233648, WO 2014 207082, WO 2014 056593, WO 2018 002319, and WO 2013 117615, or pharmaceutically acceptable salts thereof. The benzoazepine-based compound may include compounds disclosed in WO 2007 024612, WO 2010 014913, WO 2010 054215, WO 2011 022508, WO 2011 022509, WO 2012 097177, WO 2012 097173, WO 2016 096778, WO 2016 142250, WO 2017 202704, WO 2017 202703, WO 2017 216054, WO 2017 046112, and WO 2017 197624, or pharmaceutically acceptable salts thereof. The 7-thia-8-oxoguanosine-based compound may include compounds disclosed in WO 2016 180691, WO 2016 055553, WO 2016 180743, and WO 2016 091698, or pharmaceutically acceptable salts thereof. In addition, toll-like receptor 7/8 compounds disclosed in PCT/US2009/035563, PCT/US2015/028264, PCT/US2016/020499, WO 2015 023598 and PCT/US 2015/039776, or pharmaceutically acceptable salts thereof may be included. All available toll-like receptor 7/8 agonists that can be easily inferred by those of skill in the art are included.

The "toll-like receptor 7/8 agonist" used herein may be applied to a "toll-like receptor 3 agonist" or "toll-like receptor 9 agonist," which is delivered into cells and has a receptor in an endosome, in the same way, and thus the "toll-like receptor 3 agonist" or "toll-like receptor 9 agonist" may also form a complex with cholesterol.

Throughout the specification, the toll-like receptor 7/8 agonist-cholesterol complex may regulate an immune function of immune cells such that the immune activation of antigen-presenting cells (e.g. dendritic cells and macrophages), natural killer cells or T cells is induced, or regulate an immune function of immune cells by regulating the function of immune cells (regulatory T cells (Tregs), myeloid derived suppressor cells (MDSCs) and M2 macrophages), which exhibit an immunosuppressive action, in a tumor microenvironment. The function of the immune cells exhibiting an immunosuppressive action may be regulated by inhibiting the action of Tregs or MDSCs, or reducing the cell count, or regulated by a method of converting MDSCs into antigen-presenting cells inducing an anticancer immune function. Alternatively, M2 macrophages may be converted into M1 macrophages.

The "co-administration" or "combinational administration" used herein is administration of a toll-like receptor 7/8 agonist-cholesterol complex, along with various materials such as an antigen, an immune checkpoint inhibitor, an adjuvant, an immune activation material, and a chemotherapeutic agent, and there are no limitations to the type and shape of the materials.

The "chemotherapeutic agent" used herein may be any compound known to the art, which can be used in cancer treatment, without limitation, and the chemotherapeutic agent may be paclitaxel, docetaxel, 5-flurouracil, alendronate, doxorubicin, simvastatin, hydrazinocurcumin, amphotericin B, ciprofloxacin, rifabutin, rifampicin, efavirenz, cisplatin, theophyline, pseudomonas exotoxin A, zoledronic acid, trabectedin, siltuximab, dasatinib, sunitinib, apatinib, 5,6-dimethylxanthenone-4-acetic acid, silibinin, PF-04136309, trabectedin, carlumab, BLZ945, PLX3397, emactuzumab, AMG-820, IMC-CS4, GW3580, PLX6134, N-acetyl-l-cysteine, vitamin C, bortezomib, aspirin, salicylates, indolecarboxamide derivatives, quinazoline analogues, thalidomide, prostaglandin metabolites, 2ME2, 17-AAG, camptothecin, topotecan, pleurotin, 1-methylpropyl, 2-imidazolyl disulfide, tadalafil, sildenafil, L-AME, nitroaspirin, celecoxib, NOHA, bardoxolone methyl, D,L-1-methyl-tryptophan, gemcitabine, axitinib, sorafenib, cucurbitacin B, JSI-124, anti-IL-17 antibodies, anti-glycan antibodies, anti-VEGF antibodies, bevacizumab, antracycline, tasquinimod, imatinib or cyclophosphamide.

The "immune checkpoint inhibitor" used herein includes all methods of treating cancer, which activate the immune function of immune cells to fight cancer cells, and the immune checkpoint inhibitor includes, for example, anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR or anti-TIGIT.

The immune activation material used herein includes all materials for activating immune cells, and the immune activation material is, for example, a toll-like receptor agonist, a saponin, an antiviral peptide, an inflammasome inducer, a NOD ligand, a cytosolic DNA sensor (CDS) ligand, a stimulator of interferon gene (STING) ligand, an emulsion or alum.

The "antigen" used herein includes all materials causing an immune response in the body, and is preferably a pathogen (bacteria, a virus, et. al.), a chemical compound, pollen, cancer cells, shrimp, or a peptide or protein derived from the antigen, and more preferably, a cancer antigen peptide or a material that can cause an immune response in the body. The antigen is preferably a protein, a recombinant protein, a glycoprotein, a gene, a peptide, a polysaccharide, a lipopolysaccharide, a polynucleotide, a cell, a cell lysate, a bacterium or a virus, and more preferably, a cancer antigen peptide. The glycoprotein may be an antibody, an antibody fragment, a structural protein, a regulatory protein, a transcription factor, a toxin protein, a hormone, a hormone derivative, an enzyme, an enzyme fragment, a transport protein, a receptor, a receptor fragment, a biodefense inducer, a storage protein, a movement protein, an exploitive protein, or a reporter protein. However, if it is a material that can induce an immune response by acting as an antigen in the body, it is not limited thereto.

The "vaccine" used herein refers to a biological preparation containing an antigen provoking an immune response in the body, and an immunogen which creates immunity in the body by injection or oral administration to a human or animal for the prevention of infection. The animal may be a human or a non-human animal such as a pig, a cow, a horse, a dog, a goat, or a sheep.

The "prevention" used herein refers to all actions of inhibiting a disease such as cancer, an immune disease or an infectious disease or delaying the onset thereof by administration of the composition according to the present invention.

The "treatment" used herein refers to all actions involved in alleviating or beneficially changing symptoms of a disease such as cancer, an immune disease or an infectious disease by administration of the composition according to the present invention.

The "individual or subject" used herein refers to a target to which the composition of the present invention may be administered, and there is no limitation to the subject.

The "cancer" used herein refers to the generic term for various types of blood cancer and malignant solid tumors, which can expand locally by infiltration and systemically through metastasis. Although not particularly limited thereto, specific examples of cancer include colorectal cancer, adrenal cancer, bone cancer, brain cancer, breast cancer, bronchial cancer, colon cancer and/or rectal cancer, gallbladder cancer, gastrointestinal cancer, head and neck cancer, kidney cancer, laryngeal cancer, liver cancer, lung cancer, neural tissue cancer, pancreatic cancer, prostate cancer, parathyroid cancer, skin cancer, stomach cancer and thyroid cancer. Other examples of cancer include adenocarcinoma, adenoma, basal cell carcinoma, cervical dysplasia and carcinoma in situ, Ewing's sarcoma, squdamous cell carcinoma, ductal carcinoma, malignant brain tumor, hairy-cell tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, medullary carcinoma, metastatic skin cancer, mucosal neuroma, myelodysplastic syndrome, myeloma, mycosis fungoides, neuroblastoma, osteosarcoma, osteogenic and other sarcomas, ovarian cancer, pheochromocytoma, polycythemia, primary brain tumor, small-cell lung cancer, ulcerative and papillary squamous cell carcinoma, seminoma, soft tissue sarcoma, retinoblastoma, rhabdomyosarcoma, renal cell tumor or renal cell carcinoma (RCC), reticulum cell sarcoma, and Wilms' tumor. Examples of cancer also include astrocytoma, gastrointestinal stromal tumor (GIST), glioma or glioblastoma, hepatocellular carcinoma (HCC), and pancreatic neuroendocrine cancer.

The term "infectious disease" includes all diseases induced by infection caused by heterogenous organisms such as bacteria and viruses.

The "pharmaceutical composition" or "vaccine composition" used herein is prepared in the form of a capsule, tablet, granule, injection, ointment, powder or beverage, and the pharmaceutical composition or vaccine composition may be targeted at a human. The pharmaceutical composition or vaccine composition may be used in an oral formulation such as a powder, granule, capsule, tablet or an aqueous suspension, externals, a suppository, and a sterile injectable solution according to a conventional method. The pharmaceutical composition or vaccine composition of the present invention may include a pharmaceutically acceptable carrier. As pharmaceutically acceptable carriers, a binder, a lubricant, a disintegrant, an excipient, a solubilizer, a dispersant, a stabilizer, a suspending agent, a color and a flavor may be used for oral administration, a mixture of a buffer, a preservative, a pain relief agent, a solubilizer, an isotonic agent and a stabilizer may be used for an injectable, and a base, an excipient, a lubricant and a preservative may be used for local administration. The pharmaceutical composition or vaccine composition of the present invention may be prepared in various dosage forms by mixing the above-described pharmaceutically acceptable carriers. For example, for oral administration, the pharmaceutical composition or vaccine composition of the present invention may be prepared in various dosage forms such as a tablet, a troche, a capsule, an elixir, a suspension, a syrup and a wafer, and for injectables, the pharmaceutical composition or vaccine composition of the present invention may be prepared in a unit dose ampoule or multiple dose forms. In addition, the pharmaceutical composition or vaccine composition of the present invention maybe formulated as a solution, a suspension, a tablet, a capsule or a sustained-release preparation.

Meanwhile, examples of carriers, excipients and diluents suitable for preparation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. The examples of carriers, excipients and diluents may also include a filler, an anti-agglomerate, a glidant, a wetting agent, a fragrance, an emulsifier, and a preservative.

Administration routes for the pharmaceutical composition or vaccine composition according to the present invention may include oral, intravenous, intramuscular, intraarterial, intramedullary, intrathecal, intracardiac, transdermal, subcutaneous, intraperitoneal, intranasal, intraperitoneal, intranasal, intestinal, local, sublingual or rectal administration. Oral or parenteral administration is preferable. The term "parenteral" used herein means subcutaneous, intracutaneous, intravenous, intramuscular, intraarticular, intrabursal, intrasternal, intrathecal, intralesional and intracranial injection techniques. The pharmaceutical composition or vaccine composition of the present invention may be administered in the form of a suppository for rectal administration.

The pharmaceutical composition or vaccine composition of the present invention may be changed variously according to various factors including the activity of a specific compound used, age, body weight, general health, sex, diet, administration time, administration route, excretion rate, drug formulation, and the severity of a specific disease to be prevented or treated, and a dosage of the pharmaceutical composition may be suitably selected by those of ordinary skill in the art depending on a patient's condition, body weight, the severity of a disease, a drug type, an administration route and an administration duration, and may be 0.0001 to 500 mg/kg or 0.001 to 500 mg/kg per day. The pharmaceutical composition of the present invention may be administered once a day or several times in divided portions. The pharmaceutical composition or vaccine composition according to the present invention may be formulated as a pill, a caplet, a capsule, a liquid, a gel, a syrup, a slurry or a suspension.

In addition, the vaccine composition according to the present invention may further include a conventionally known "adjuvant". The adjuvant generally encompasses all materials increasing humoral and/or cellular immune response(s) against an antigen, and any adjuvant known in the art can be used without limitation. For example, immunity may be increased by further adding a Freund's complete or incomplete additive to the vaccine composition according to the present invention. In addition, in the case of the vaccine composition, if necessary, optionally repeated antigen stimulation may be performed after an initial dose.

Hereinafter, to help in understanding the present invention, exemplary examples will be suggested. However, the following examples are merely provided to more easily understand the present invention, and not to limit the present invention.

### [Examples]

### Example 1: Synthesis of toll-like receptor 7/8 agonist-cholesterol complex

Various toll-like receptor 7/8 agonists (imidazoquinoline-based, 8-hydroxyadenine-based, pteridone-based, 2-aminopyrimidine-based, benzoazepine-based, and 7-thia-8-oxoguanosine-based) conjugated to cholesterol were prepared by a chemical reaction such as Reaction Scheme 1 or 2. The toll-like receptor 7/8 agonist may be prepared by reacting an active site of the toll-like receptor 7/8 agonist, an amine group (NH₂), with a cholesterol derivative that can form a bond with a carbamate, a disulfide, an ester, a peptide or an azide. The derivative encompasses similar compounds obtained by chemically changing a part of a toll-like receptor 7/8 agonist. 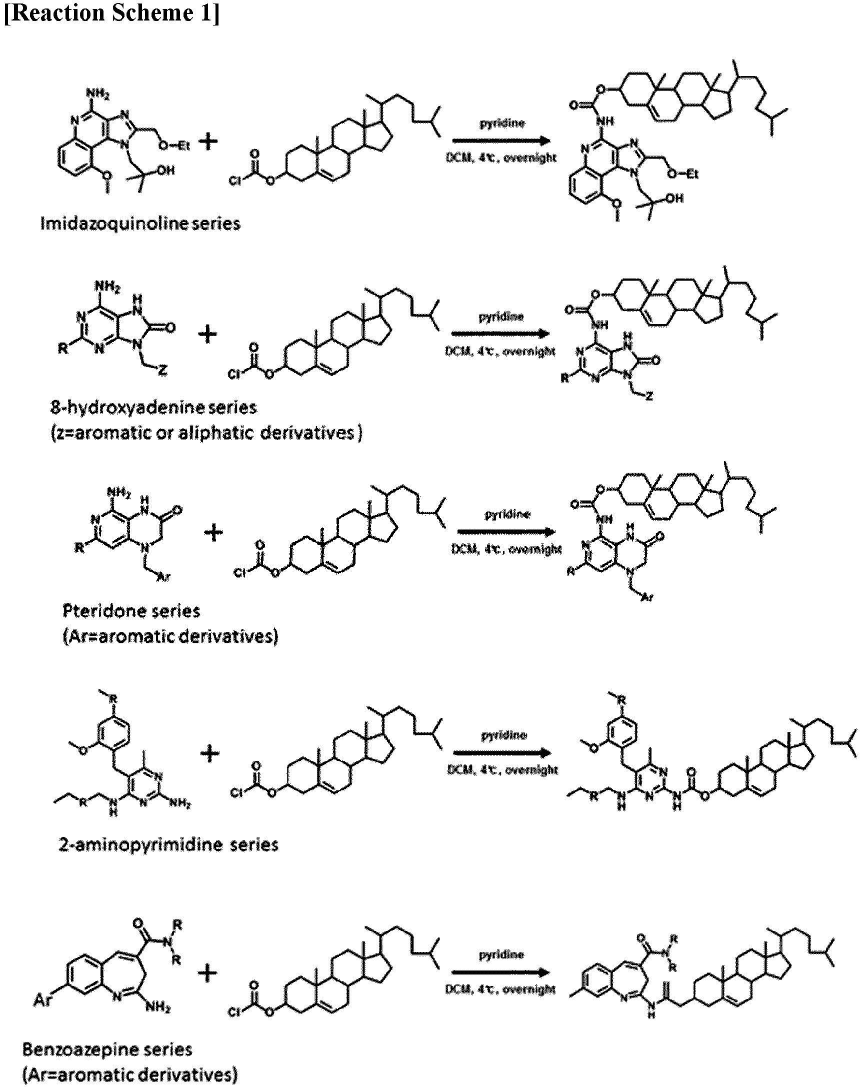
R is a side chain having an aliphatic or aromatic group, and may include -NH-, -CO-, -CONH-, -CSNH-, -COO-, -CSO-, -SO₂NH-, -SO₂-, -SO-, and -O-. 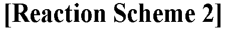 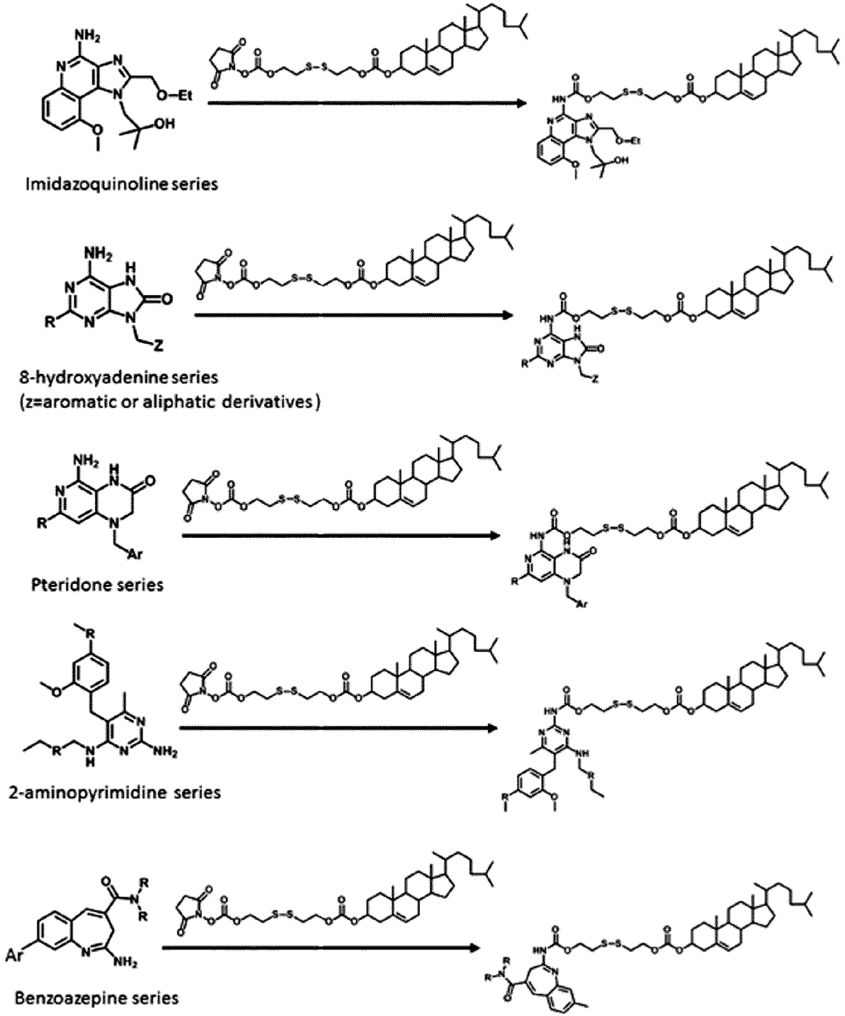
R is a side chain having an aliphatic or aromatic group, and may include -NH-, -CO-, -CONH-, -CSNH-, -COO-, -CSO-, -SO₂NH-, -SO₂-, -SO-, and -O-.

### 1.1. Synthesis of resquimod-cholesterol complex having carbamate bond

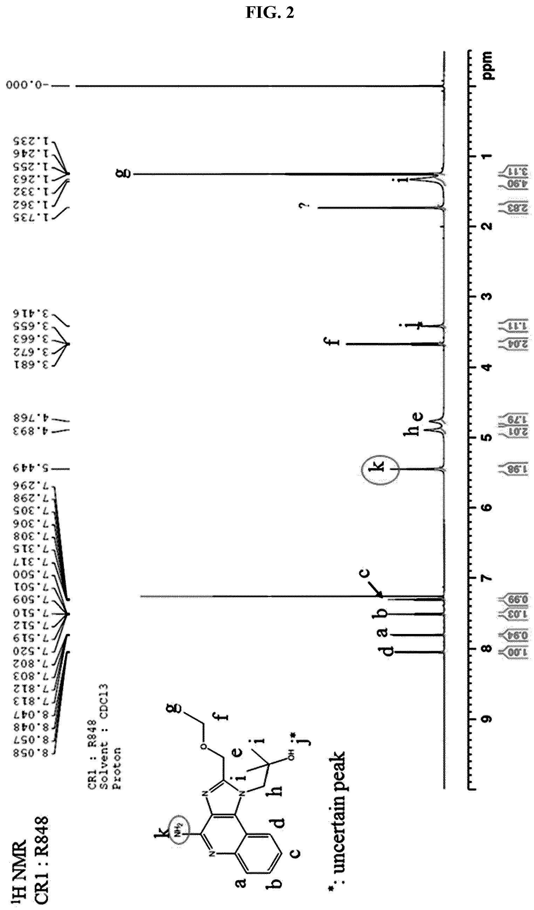

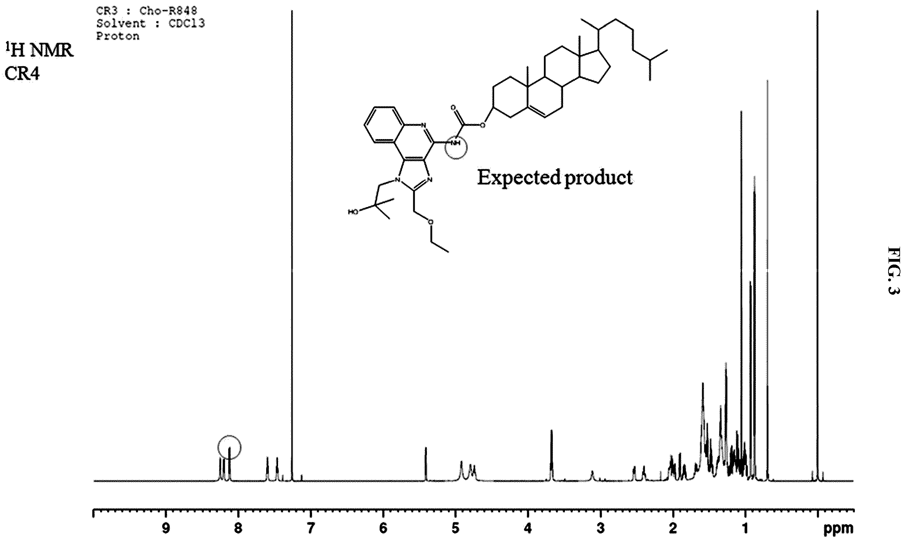

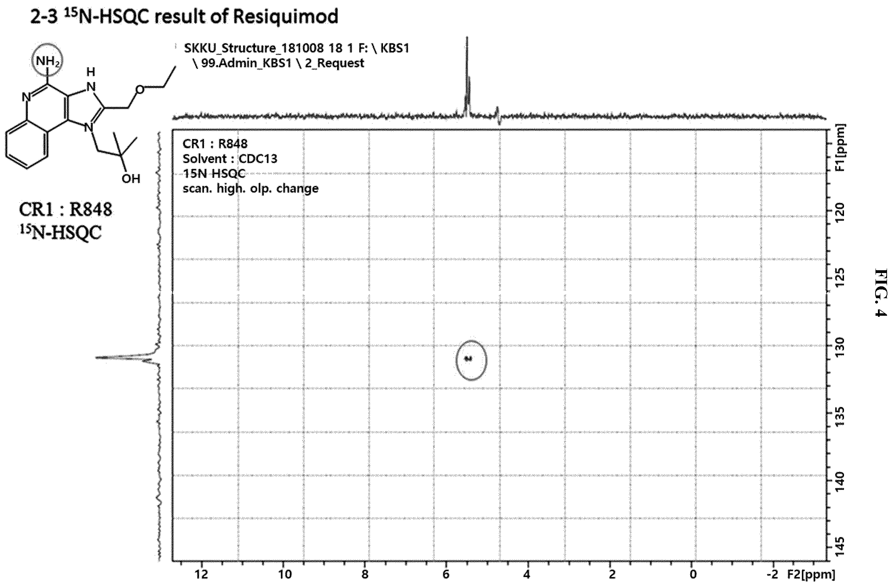

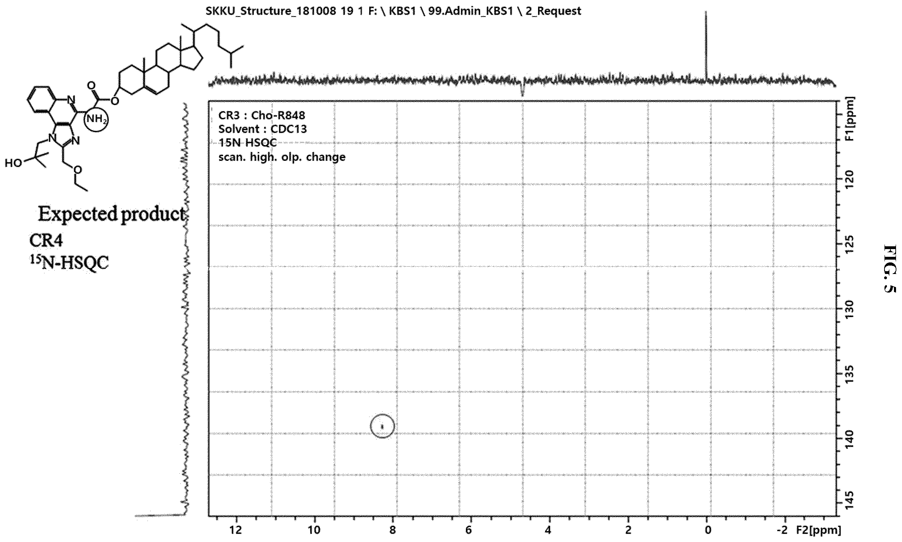

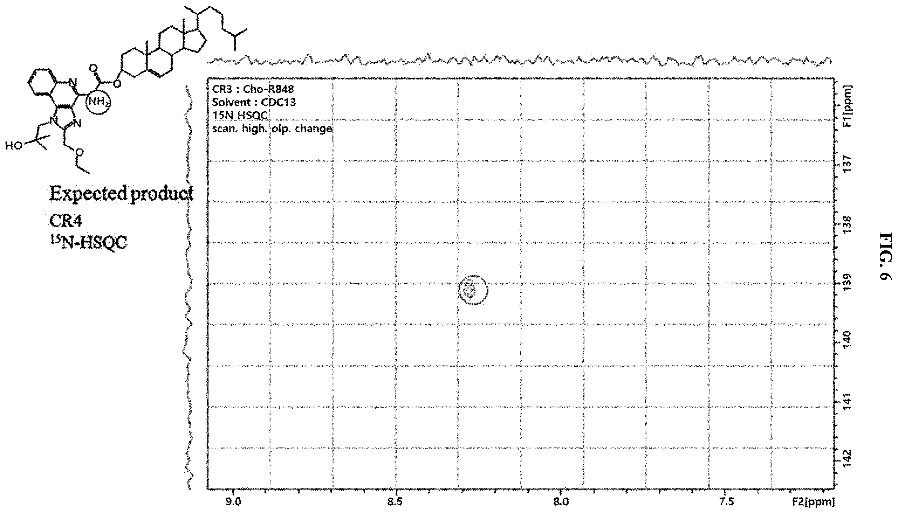

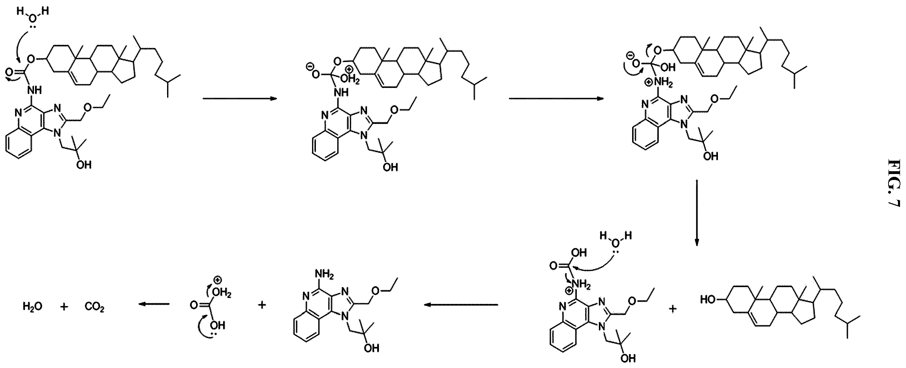

To synthesize cholesterol-conjugated resquimod (R848), a method in Reaction Scheme 3 below was used. More specifically, a solution in which 90.0 mg of cholesteryl chloroformate was added and dissolved in 1 mL of dichloromethane was slowly added dropwise to a solution in which 100 µL of pyridine was added to 31.4 mg of resquimod, and added and dissolved in 3 mL of dichloromethane. Subsequently, a mixture was prepared by stirring the resulting solution at 4 °C for 16 hours. In addition, the mixture was adjusted to room temperature, and distilled water was added to separate water and a dichloromethane layer. Subsequently, sodium sulfate was added to the separated dichloromethane layer, and reacted for 16 hours to remove remaining water. In addition, the remaining solution was purified using a silica gel column, thereby obtaining white cholesterol-conjugated resquimod powder. The structures of resquimod used in synthesis and the obtained cholesterol-conjugated resquimod were verified using ¹H-NMR and ¹⁵N-heteronuclear single quantum coherence (HSQC) spectroscopy. The structures of resquimod are shown in FIGS. 2 and 4, and the structures of cholesterol-conjugated resquimod are shown in FIGS. 3, 5 and 6. A separating mechanism of cholesterol from the cholesterol-conjugated resquimod due to a physiological environment in cells is shown in FIG. 7.

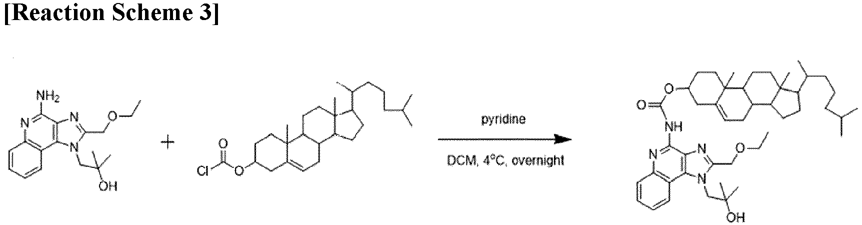

### 1.2. Synthesis of imiquimod-cholesterol complex having carbamate bond

To synthesize cholesterol-conjugated imiquimod (R837), a method shown in Reaction Scheme 4 below was used. More specifically, a solution in which 90.0 mg of cholesteryl chloroformate was added and dissolved in 1 mL of dichloromethane was slowly added dropwise to a solution in which 100 µL of pyridine was added to 31.4 mg of imiquimod, and then added and dissolved in 3 mL of dichloromethane. Subsequently, a mixture was prepared by stirring the resulting solution at 4 °C for 16 hours. In addition, the mixture was adjusted to room temperature, and then distilled water was added to separate water and a dichloromethane layer, and sodium sulfate was added to the separated dichloromethane layer to allow a reaction for 16 hours so as to remove remaining water. The remaining solution was then purified using a silica gel column, thereby obtaining white powdery cholesterol-conjugated imiquimod. 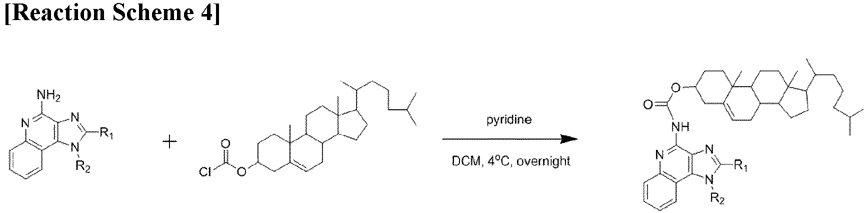 R₁ or R₂ is a side chain having an aliphatic or aromatic group, and may include -NH-, -CO-, -CONH-, -CSNH-, -COO-, -CSO-, -SO₂NH-, -SO₂-, -SO-, and -O-.

### Example 2: Synthesis of toll-like receptor 7/8 agonist-cholesterol complex crosslinked by disulfide

### 2.1. Synthesis of resquimod-cholesterol complex crosslinked by disulfide

To synthesize resquimod crosslinked with cholesterol-disulfide, a method of Reaction Scheme 5 below was used. More specifically, 1.54 g of 2-hydroxyethyl disulfide was added and dissolved in 30 mL of tetrahydrofuran, and then slowly added dropwise to a phosgene solution (15 mL, 15 wt% in toluene), thereby preparing a mixture. In addition, the mixture was stirred at 25 °C for 10 hours, and then evaporated the solvent *in vacuo.* In addition, 2.3 g of N-hydrosuccinimide was added and dissolved in tetrahydrofuran and mixed with the mixture, followed by adding 1.57 mL of triethylamine. In addition, after a reaction at 40 °C for 16 hours, a precipitate was removed, and then the solvent was evaporated *in vacuo.* In addition, the resulting solution was purified by silica gel column chromatography and recrystallized using cold hexane, and dried *in vacuo,* thereby obtaining a white solid, which is a purified disulfide-crosslinked linker. In addition, 387 mg of cholesterol was added and dissolved in 10 mL dichloromethane, 523 mg of a disulfide-crosslinked linker was added and stirred at room temperature for 16 hours, and then white cholesterol-disulfide powder in which cholesterol and disulfide are linked was purified using a silica gel column. In addition, 31.4 mg of resquimod and 80 mg of cholesterol-disulfide were added to 5 mL dichloromethane, and stirred at room temperature for 16 hours. In addition, distilled water was added to the stirred solution to separate water and a dichloromethane layer, and sodium sulfate was added to the separated dichloromethane layer and reacted for 16 hours so as to remove remaining water. In addition, the remaining solution was purified using a silica gel column, thereby obtaining white cholesterol-disulfide-crosslinked resquimod powder.

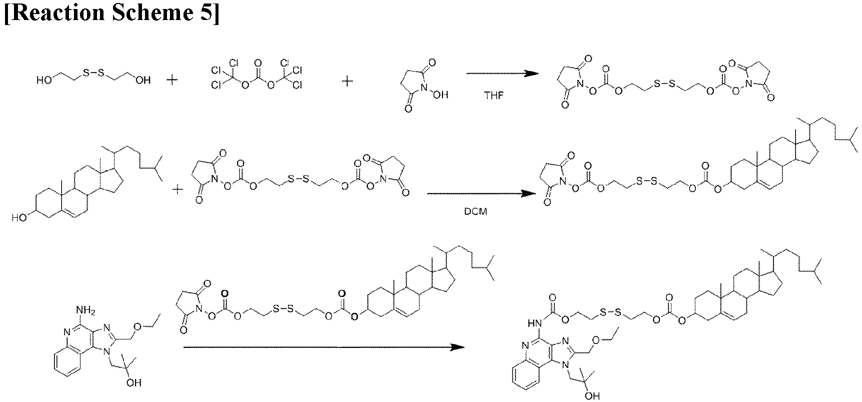

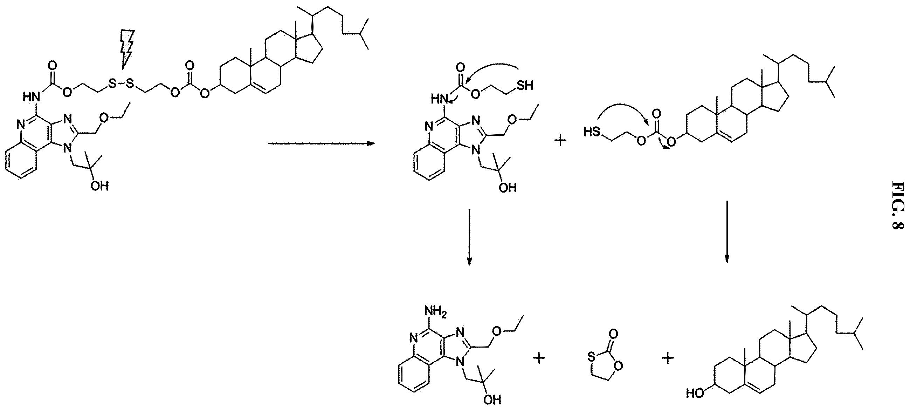

A separating mechanism of cholesterol from the cholesterol-disulfide-crosslinked resquimod due to a physiological environment in cells is shown in FIG. 8.

### 2.2. Synthesis of disulfide-crosslinked imiquimod-cholesterol complex

To synthesize cholesterol-disulfide-crosslinked imiquimod, a method of Reaction Scheme 6 was used. More specifically, 1.54 g of 2-hydroxyethyl disulfide was added and dissolved in 30 mL tetrahydrofuran, and then slowly added dropwise to a phosgene solution (15 mL, 15 wt% in toluene), thereby preparing a mixture. In addition, the mixture was stirred at 25 °C for 10 hours, and then evaporated the solvent *in vacuo.* In addition, 2.3 g of N-hydrosuccinimide was added and dissolved in tetrahydrofuran, and mixed with the mixture, followed by adding 1.57 mL triethylamine. In addition, after a reaction at 40 °C for 16 hours, a precipitate was removed, and then the solvent was evaporated *in vacuo.* In addition, the resulting solution was purified by silica gel column chromatography and recrystallized using cold hexane, and dried *in vacuo,* thereby obtaining a white solid, which is a purified disulfide-crosslinked linker. In addition, 387 mg of cholesterol was added and dissolved in 10 mL dichloromethane, 523 mg of a disulfide-crosslinked linker was added and stirred at room temperature for 16 hours, and then white cholesterol-disulfide powder in which cholesterol and disulfide were conjugated was purified using a silica gel column. In addition, 31.4 mg of imiquimod and 80 mg of cholesterol-disulfide were added to 5 mL dichloromethane, and stirred at room temperature for 16 hours. In addition, distilled water was added to the stirred solution to separate water and a dichloromethane layer, and sodium sulfate was added to the separated dichloromethane layer and reacted for 16 hours to remove remaining water. In addition, the remaining solution was purified using a silica gel column, thereby obtaining white cholesterol-disulfide-crosslinked imiquimod powder. 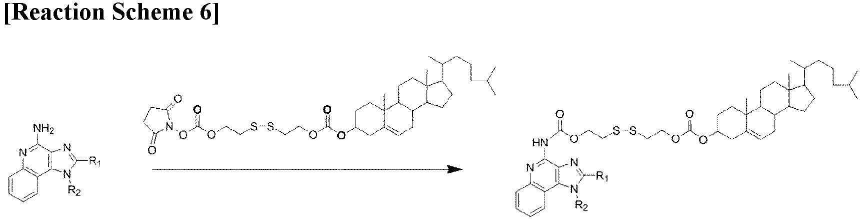 R₁ or R₂ is a side chain having an aliphatic or aromatic group, and may include -NH-, -CO-, -CONH-, -CSNH-, -COO-, -CSO-, -SO₂NH-, -SO₂-, -SO-, and -O-.

### Example 3: Preparation of nanoparticles including cholesterol-toll-like receptor 7/ 8 agonist complex

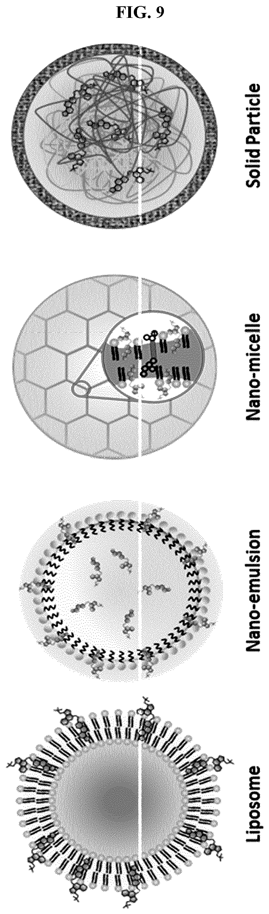

To maximize the interaction with immune cells, a cholesterol-toll-like receptor 7/8 agonist complex may be prepared in various forms of nanoparticles (FIG. 9).

### 3.1. Preparation of nanoliposome including cholesterol-conjugated resquimod

To prepare an anionic nanoliposome including cholesterol-conjugated resquimod, 4 mg of 1,2-dioleoyl-sn-glycero-3-phosphocholine (DOPC, Avanti), 1.2 mg of cholesterol-conjugated resquimod, and 1 mg of 1,2-dipalmitoyl-sn-glycero-3-phospho-(1'-rac-glycerol) (DPPG, Avanti) were added and dissolved in 1 mL of chloroform, thereby preparing a mixture. The mixture was prepared in a thin film form by evaporating the solvent using a rotary evaporator, and 2 mL of phosphate-buffered saline was added to the thin film, and stirred at 45 °C for 30 minutes, thereby preparing an anionic nanoliposome including cholesterol-conjugated resquimod through homogenization using a tip sonicator (amplitude: 20%, 2 min). To prepare a cationic nanoliposome including cholesterol-conjugated resquimod, 4 mg of DOPC, 1.2 mg of cholesterol-conjugated resquimod, and 2 mg of dimethyloctadecylammonium bromide (DDAB) were added and dissolved in 1 mL of chloroform, thereby preparing a mixture. The mixture was prepared in a thin film form by evaporating the solvent using a rotary evaporator, and 2 mL of phosphate-buffered saline was added to the thin film, and stirred at 45 °C for 30 minutes, thereby preparing a cationic nanoliposome including cholesterol-conjugated resquimod through homogenization using a tip sonicator (amplitude: 20%, 2 min).

### 3.2. Preparation of nanoemulsion including cholesterol-conjugated resquimod

To prepare a nanoemulsion including cholesterol-conjugated resquimod, 1 mg of DOPC, 240 µg of cholesterol, and 240 µg of cholesterol-conjugated resquimod were added and dissolved in 1 mL of chloroform, thereby preparing a mixture. In addition, the mixture was prepared in the form of a thin lipid film by containing the mixture in a round-bottom flask and completely evaporating chloroform using a rotary evaporator. In addition, squalene (5 %v/v), Tween 80 (0.5 %v/v) and Span 85 (0.5 %v/v) were added and dissolved in 2 mL of phosphate-buffered saline, and the solution was added over the lipid film, dispersed using a tip sonicator for 1 minute, and stirred using a tube revolver for approximately 2 hours, thereby preparing a nanoemulsion including cholesterol-conjugated resquimod, and then stored at 4 °C in a refrigerator until use.

### 3.3. Preparation of nanomicelle consisting of cholesterol-conjugated resquimod and saponin

To prepare a nanomicelle consisting of cholesterol-conjugated resquimod and saponin, phosphatidylcholine, saponin and cholesterol-conjugated resquimod were mixed in a weight ratio of 5: 3: 2, and ether was added and dissolved in the mixture to have a concentration of 14 mg/mL, thereby preparing an ether solution including a lipid. In addition, saponin was dissolved in 4 mL distilled water at a concentration of 1.5 mg/mL, and contained in a 20 mL glass bottle. The glass bottle was closed with a rubber stopper, and then stored in a 55 °C water jacket. In addition, 1 mL of the lipid-containing ether solution was added to the glass bottle containing saponin at a rate of 0.2 mL/min using a syringe pump, and stirred for 2 hours. Here, the tip of a syringe needle was placed below the surface of the saponin-containing aqueous solution, and a second needle was inserted into the rubber stopper for ventilation. In addition, the glass bottle was transferred at room temperature, stirred for 3 hours to stabilize, thereby preparing a nanomicelle consisting of cholesterol-conjugated resquimod and saponin.

### 3.4. Preparation of polymer nanoparticle consisting of cholesterol-conjugated resquimod

60 mg of a PLGA polymer (Eudragit) having a composition ratio of lactide and glycolide of 50 : 50 was dissolved in 1 mL of a chloroform solvent. 5 mg of cholesterol-conjugated resquimod was added to the solvent and the cholesterol-resquimod complex and the polymer were dissolved using a sonicator (ultrasonic bath, Emerson Model CPX5800H-E). In addition, the resulting solution was added to 10 mL of a 2.5 % PVA aqueous solution by 200 µL, and dispersed for 1 minute using a tip sonicator (Sonics & Materials Model VCX 750). Here, the output of a disperser was 750 watts, a vibration intensity was 20 kHz, and an amplitude was set to 20%. In addition, to completely evaporate the PLGA-dissolved organic solvent, the prepared aqueous solution was stirred at 600 rpm and room temperature for 8 hours or more. To remove the unreacted polymer and cholesterol-resquimod complex, centrifugation was performed at 12,000 rpm for 12 minutes using a centrifuge (Hanil, Combi-514R), a supernatant was removed, 10 mL of deionized water was added, and then the resulting solution was dispersed in a sonicator for 30 seconds. The above-described process was repeated three times, followed by drying by lyophilization and storing at -20 °C.

### Example 4: Characterization of nanoparticles including cholesterol-toll-like receptor 7/8 agonist complex and evaluation of immune activating efficacy of immune cells

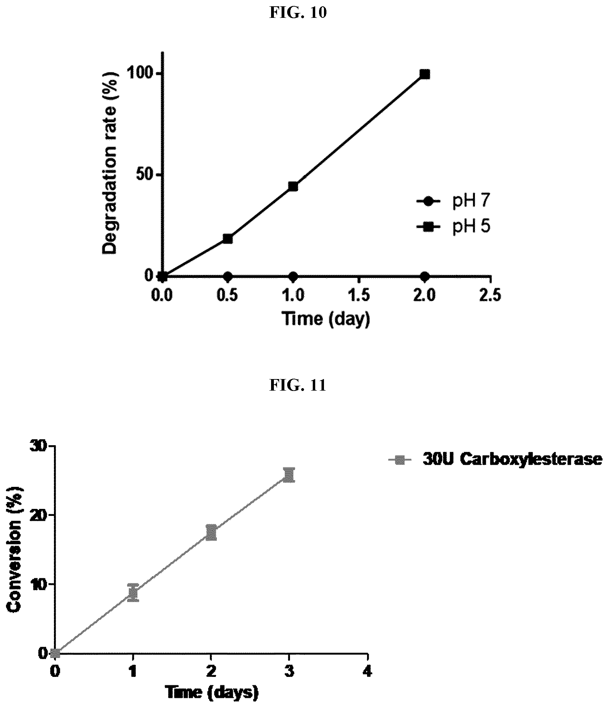

After nanoparticles including the cholesterol-toll-like receptor 7/8 agonist complex of the present invention were delivered into cells, it was confirmed whether cholesterol and resquimod were separated under acidic conditions in the cytoplasm. More specifically, nanoliposomes including cholesterol-conjugated resquimod were prepared by the same method as in Example 3.1, 10 µg of the prepared nanoliposome was added to 1 mL of phosphate-buffered saline (PBS) having pH 5 or 7 at 37 °C. In addition, each sample was obtained on day 0.5, 1, 1.5 or 2 to measure a concentration of separated resquimod using a UV-Vis spectrum. The result is shown in FIG. 10.

As shown in FIG. 10, while resquimod and cholesterol were not separated at pH 7, it was confirmed that resquimod is separated from cholesterol over time at pH 5. According to the result, after the nanoparticles including the cholesterol-toll-like receptor 7/8 agonist complex were delivered into the cytoplasm in the body, it was confirmed that the toll-like receptor 7/8 agonist is separated from cholesterol to induce an immune activating reaction in the cytoplasm.

In addition, to confirm whether cleavage is made by a specific enzyme present in a tumor microenvironment excluding pH, a nanoliposome including cholesterol-conjugated resquimod was prepared by the same method as Example 3.1, 30 units of carboxylesterase were treated and reacted at 37 °C. In addition, cholesterol cleavage was confirmed. The result is shown in FIG. 11.

As shown in FIG. 11, it was confirmed that the cholesterol of the cholesterol-toll-like receptor 7/8 agonist complex was cleaved by a specific enzyme present in cells of a tumor microenvironment.

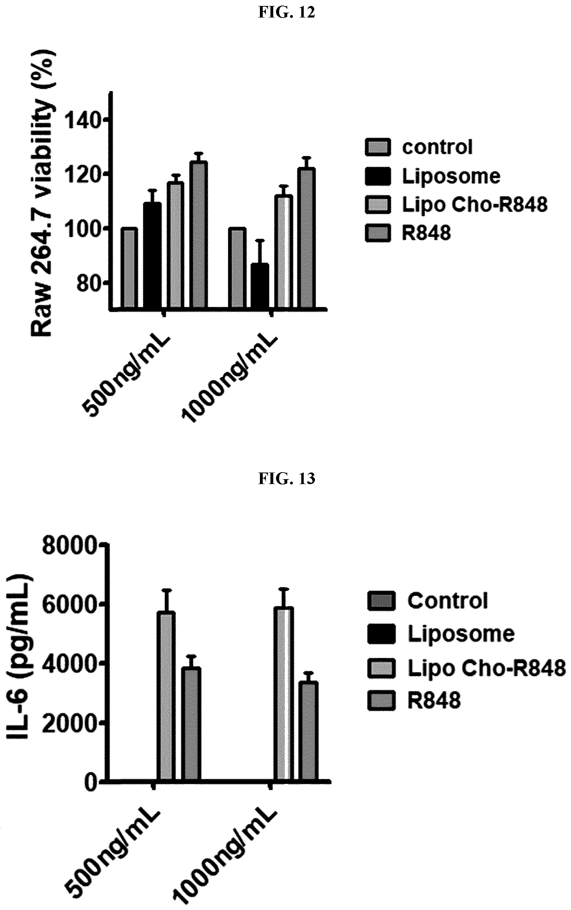

To confirm cytotoxicity, 10⁴ Raw264.7 macrophages were treated with 100 µL each of 500 ng/mL and 1,000 ng/mL of liposomes, nanoliposomes including a cholesterol-resquimod complex, and resquimod, and reacted for 24 hours. In addition, a cell survival rate was measured using Cell Titer 96 AQueous One Solution Cell Proliferation Assay (Promega). As a negative control, an experiment was carried out using phosphate-buffered saline. The result is shown in FIG. 12.

As shown in FIG. 12, it was confirmed that the nanoliposomes including the cholesterol-resquimod complex do not only exhibit cytotoxicity, but also exhibit an effect of promoting proliferation by immune cell activation.

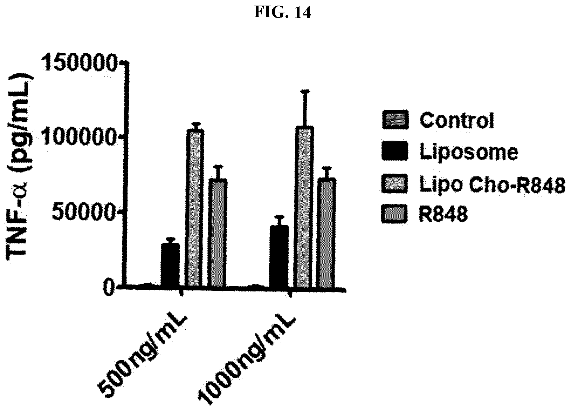

In addition, to confirm the degree of immune cell activation, 10⁶ Raw264.7 macrophages were treated with 1 mL each of 500 ng/mL and 1,000 ng/mL of liposomes, nanoliposomes including a cholesterol-resquimod complex and resquimod, and incubated for 24 hours, thereby obtaining a liquid cell culture. The liquid cell culture was centrifuged at 1,500 rpm for 10 minutes to obtain a cell-free supernatant, and the concentrations of IL-6 and TNF-α included in the supernatant were measured through ELISA using a BD OptiEIATM kit. The result is shown in FIGS. 13 and 14.

As shown in FIGS. 13 and 14, compared to when resquimod was treated alone, it was confirmed that IL-6 and TNF-α release amounts increase in an experimental group treated with the nanoliposomes including a cholesterol-resquimod complex, demonstrating that the immune activation of a toll-like receptor 7/8 agonist may not only be stably induced at a desired point of time in cells, but also increase the degree of immune activation, using the nanoliposomes including a cholesterol-toll-like receptor 7/8 agonist complex.

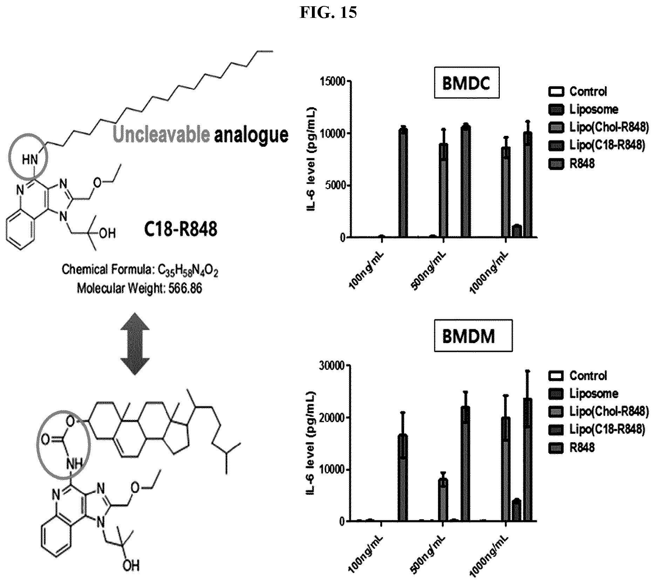

The degree of the activation of immune cells was confirmed using a cholesterol-resquimod complex in which cholesterol is not cleaved at a specific point of time as a control. More specifically, 2×10⁵ cells/mL of bone marrow-derived dendritic cells (BMDCs) or bone marrow-derived macrophages (BMDMs) were treated with each of a liposome (Lipo(Chol-R848)) including the cholesterol-resquimod complex of the present invention, a liposome (Lipo(C18-R848)) including an uncleaved cholesterol-resquimod complex, a liposome and resquimod by concentration, and incubated for 24 hours. In addition, a liquid culture was obtained, centrifuged at 490 g for 5 minutes, thereby obtaining only a supernatant, and ELISA was carried out using a BD OptEIA^{™} kit. The result is shown in FIG. 15.

As shown in FIG. 15, while the cholesterol-toll-like receptor 7/8 agonist complex of the present invention effectively activates immune cells by inducing the cleavage of cholesterol in the cytoplasm, it was confirmed that the complex in which cholesterol cleavage is not induced does not activate immune cells since the active site of resquimod is not exposed. From the above results, it was confirmed that the cholesterol-toll-like receptor 7/8 agonist complex of the present invention is inactivated when cholesterol is not cleaved, but effectively activates immune cells after cholesterol is cleaved under a specific condition, thereby regulating an immune response.

### Example 5: Evaluation of immune activation in lymph node and toxic effect in serum after in vivo injection of cholesterol-toll-like receptor 7/8 agonist complex

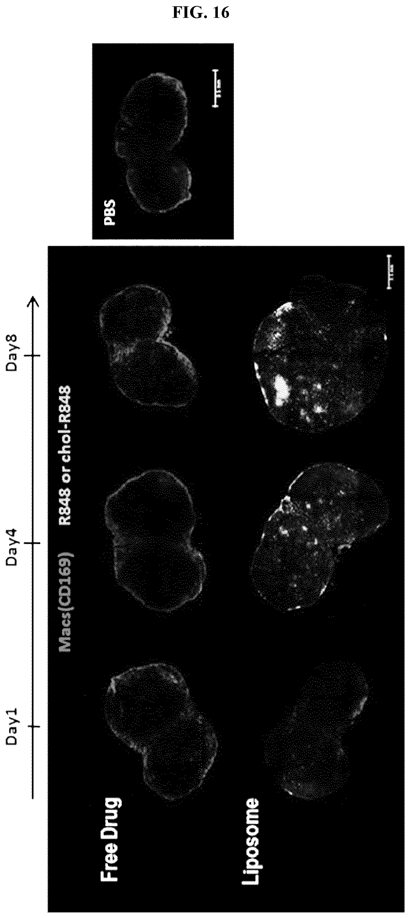

To confirm an effect of the cholesterol-toll-like receptor 7/8 agonist complex *in vivo,* an *in vivo* experiment was carried out. More specifically, a liposome including a cholesterol-resquimod complex or resquimod (free drug) was subcutaneously injected into the right flank of a C57BL/6 mouse at 25 µg/100 µL. In addition, after 1, 4 and 8 days, a lymph node was separated from the mouse, followed by cryosection. In addition, dendritic cells and macrophages were labeled with a CD205 antibody and CD169 antibody, respectively, which was detected using a fluorescent microscope. The result is shown in FIG. 16.

As shown in FIG. 16, while the activation of immune cells in a lymph node is not induced in the case of mice treated with resquimod alone, it was confirmed that immune cells are activated in a lymph node in the case of mice treated with the liposome including the cholesterol-resquimod complex.

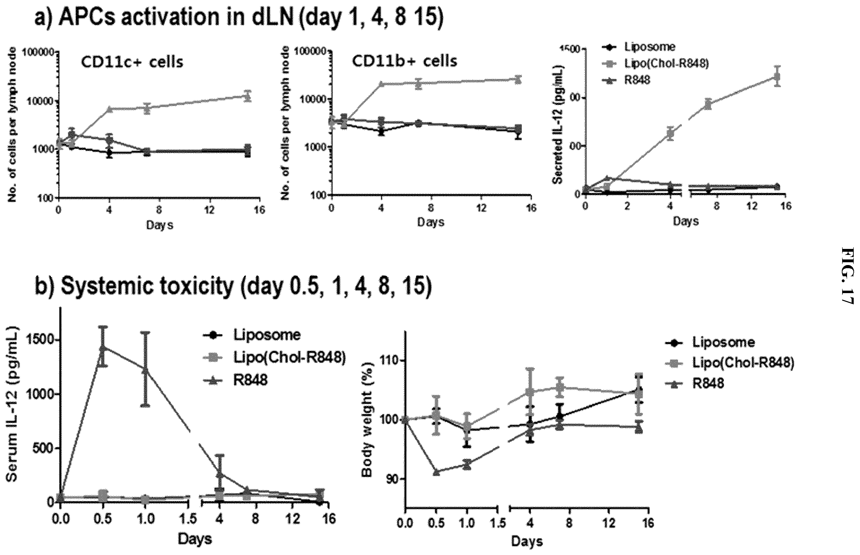

In addition, to confirm the long-term *in vivo* effect of the cholesterol-toll-like receptor 7/8 agonist complex, a liposome (Lipo(Chol-R848)) including the cholesterol-resquimod complex, a liposome or resquimod (R848) was subcutaneously injected into the right flank of C57BL/6 mice at 25 µg/100 µL, and then lymph nodes were obtained according to treatment time until 15 days. In addition, the obtained lymph nodes were first physically fragmented using scissors, treated with 1 mg/mL of collagenase D, reacted for 1 hour for second lysis, and then filtered using a 70 µm strainer, followed by separating the cells and a supernatant using a centrifuge (490 g, 5 min). In addition, the obtained cells were labeled with a CD11c antibody and CD11b antibody, and fixed using 4% paraformaldehyde. In addition, analysis was carried out using a BD FACS Canto II flow cytometer. In addition, the separated supernatant was analyzed through ELISA to measure an amount of cytokine IL-12. The result is shown in FIG. 17A.

As shown in FIG. 17A, while the activation of immune cells was not induced in mice treated with a liposome or resquimod alone, it was confirmed that in the experimental group treated with nanoparticles including a cholesterol-toll-like receptor 7/8 agonist complex of the present invention, not only the number of immune cells in a lymph node gradually increased, and immune cells were effectively activated, thereby stably releasing a cytokine for a long time.

In addition, to confirm toxicity *in vivo,* a liposome (Lipo(Chol-R848)) including the cholesterol-resquimod complex, a liposome or resquimod (R848) was subcutaneously injected into the right flank of a C57BL/6 mouse at 25 µg/100 µL, the body weight of the mouse was measured, and blood was collected from the orbital vein on each time. In addition, the collected blood was centrifuged (4 °C, 13,000 rpm, 20 min) to separate serum and blood cells, and a cytokine amount was measured through ELISA using the serum. The result is shown in FIG. 17B.

As shown in FIG. 17B, in the experimental group treated with resquimod alone, a cytokine rapidly increased in serum at an early stage to induce a cytokine storm, thereby dramatically changing a body weight by inducing cytotoxicity, whereas in the case of a nanoparticle including a cholesterol-toll-like receptor 7/8 agonist complex of the present invention, a cytokine amount was not changed in serum, and a body weight loss was not observed, confirming no *in vivo* toxicity.

### Example 6: Verification of anticancer effect of cholesterol-toll-like receptor 7/8 agonist complex

### 6.1. Verification of anticancer effect of cholesterol-toll-like receptor 7/8 agonist complex and cancer antigen

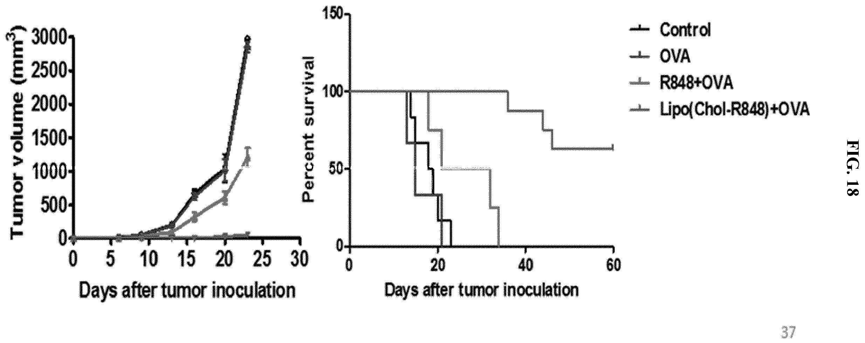

To confirm that the cholesterol-toll-like receptor 7/8 agonist complex exhibits an anticancer effect, an experiment was carried out using a B 16-OVA melanoma cell line and an ovalbumin (OVA) antigen. More specifically, 5×10⁵ cells of a B16-OVA cell line were subcutaneously injected into the right flank of a C57BL/6 mouse, and the time when a tumor size reached approximately 50 mm³ was set to day 0, each of phosphate-buffered saline (control), an OVA antigen (OVA), resquimod + OVA antigen (R848+OVA), and a nanoliposome including a cholesterol-resquimod complex, + OVA antigen (Lipo(Chol-R848)+OVA) were administered into a mouse through intratumoral injection on day 0, 3, 6 and 9. 10 µg of the OVA antigen was administered, 25 µg of resquimod was administered, and 25 µg (based on resquimod) of the nanoliposome including a cholesterol-resquimod complex was administered. In addition, a mouse survival rate and a cancer size were measured. The result is shown in FIG. 18.

As shown in FIG. 18, in the experiment group injected only with the OVA antigen, like the control injected with phosphate-buffered saline, a cancer size dramatically increased, and there was no mouse surviving three weeks or more, and in the experimental group injected with resquimod and an OVA antigen, compared to the control, it was confirmed that a rate of increase in the cancer size is reduced, but the cancer size still increases. However, in the experimental group injected with the nanoparticle including the cholesterol-toll-like receptor 7/8 agonist complex of the present invention, it was confirmed that cancer hardly grows, and even after 60 days, a survival rate of approximately 60% or more is shown.

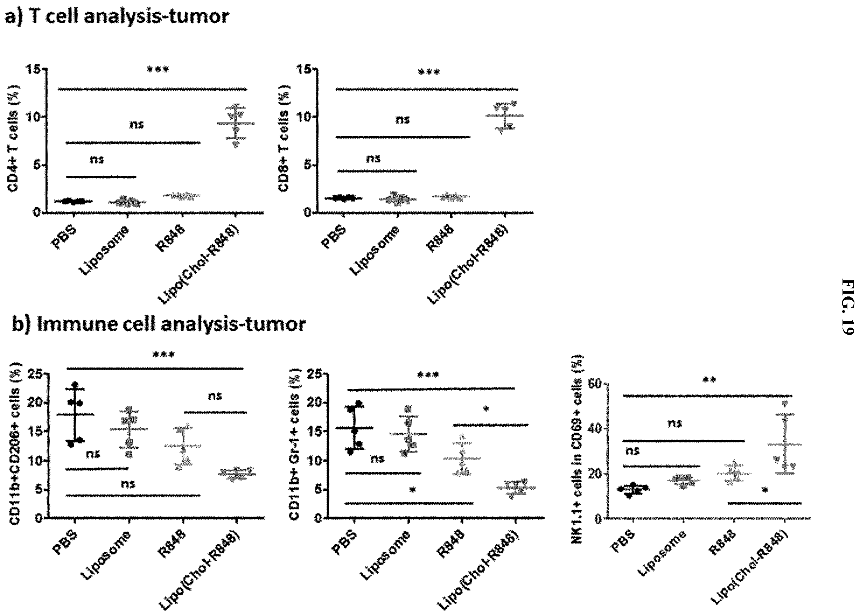

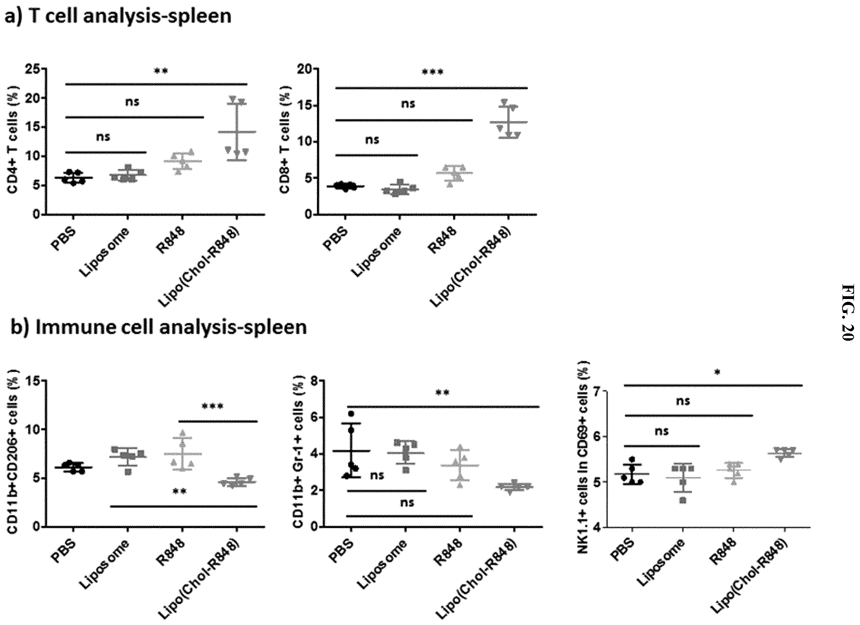

In addition, to confirm an effect on immune cells, in the same manner as above, each of PBS, an OVA antigen and nanoliposome (liposome), resquimod and an OVA antigen (R848), and a nanoliposome including a cholesterol-resquimod complex and an OVA antigen (Lipo(Chol-R848)) were administered on day 0, 3, 6 and 9 through intratumoral injection, and 7 days after administration of the final sample, tumor tissue and a spleen were separated from a mouse. In addition, the obtained tumor tissue was primarily fragmented using scissors, the fragmented tissue was treated with 1 mg/mL of collagenase type I, and reacted at 37 °C for 1 hour to separate into single cells. In addition, the separated cells were filtered using a 70 µm strainer, and washed using phosphate-buffered saline. The obtained spleen was primarily fragmented using scissors, treated with a red blood cell lysis buffer, and reacted at 37 °C for 10 minutes to lyse red blood cells. In addition, the cells were filtered using a 70 µm strainer, and washed with phosphate-buffered saline. The washed tumor cells and spleen cells were labeled with antibodies. T cells were labeled with anti-CD4 antibody and anti-CD8 antibody, type 2 macrophages (M2 macrophages) were labeled with anti-CD11b antibody and anti-CD206 antibody, myeloid-derived suppressor cells (MDSCs) were labeled with anti-CD11b antibody and anti-Gr1 antibody, and natural killer cells (NK cells) were labeled with anti-NK1.1 antibody. In addition, these cells were analyzed using a fluorescence flow cytometer. The result is shown in FIGS. 19 and 20.

As shown in FIGS. 19 and 20, while, in the experimental group injected with the nanoparticle including a cholesterol-toll-like receptor 7/8 agonist complex, the numbers of CD4+ T cells, CD8+ T cells and NK cells, which exhibit an anticancer effect in tumor tissue, significantly increase, it was confirmed that the numbers of M2 macrophages and MDSC cells, which have an immunosuppressive function, are significantly reduced.

### 6.2. Verification of anticancer effect caused by co-administration of cholesterol-toll-like receptor 7/8 agonist complex and immune checkpoint inhibitor

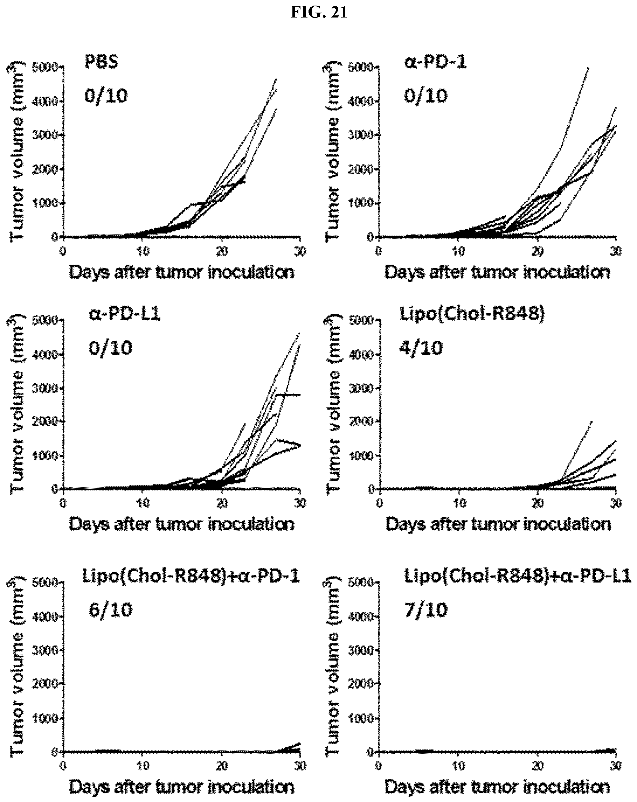

To confirm an anticancer effect of co-treatment of the cholesterol-toll-like receptor 7/8 agonist complex and an immune checkpoint inhibitor, an experiment was carried out with a B16-OVA melanoma cell line, a TC-1 lung cancer cell line, and a 4T1 breast cancer cell line. More specifically, 5×10⁵ cells of the B16-OVA cell line, TC-1 cell line or 4T1 cell line were subcutaneously injected into the right flank of a C57BL/6 mouse, and after the time when the tumor size reached approximately 50 mm³ was set to day 0, phosphate-buffered saline (PBS); anti-PD-1 and a cancer antigen (α-PD-1); anti-PD-L1 and a cancer antigen (α-PD-L1); a nanoliposome including a cholesterol-resquimod complex and a cancer antigen (Lipo(Chol-R848)); a nanoliposome including a cholesterol-resquimod complex, anti-PD-1 and a cancer antigen (Lipo(Chol-R848)+α-PD-1); and a nanoliposome including a cholesterol-resquimod complex, anti-PD-L1 and a cancer antigen (Lipo(Chol-R848)+α-PD-L1) were administered on day 0, 3, 6 and 9. Anti-PD-1 and anti-PD-L1, which are immune checkpoint inhibitors, were intraperitoneally injected at 100 µg/100 µL on day 0, 3 and 6, and the others were administered through intratumoral injection in the same manner as in Example 5.1. As the cancer antigen, an OVA antigen was used for a B16-OVA animal model, a peptide-based antigen was used for a TC-1 animal model, and a tumor cell lysate was used as an antigen for a 4T1 animal model. In addition, a mouse survival rate and a cancer size were measured. The results are shown in FIGS. 21 to 23.

As shown in FIG. 21, in the case of the B16-OVA animal model, it was confirmed that tumor growth is considerably inhibited, and 6 or 7 tumor-free mice among 10 mice are observed in the experimental group co-administered an immune checkpoint inhibitor, indicating a high anticancer effect.

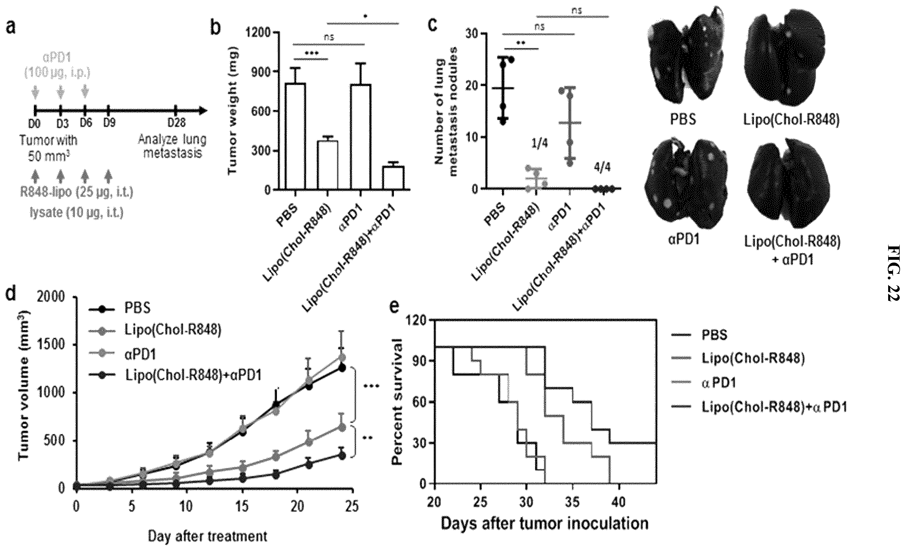

As shown in FIG. 22, in the case of the TC1 animal model, it was also confirmed that tumor growth is considerably inhibited and a survival rate is increased in the experimental group co-administered an immune checkpoint inhibitor. Moreover, it was confirmed that metastasis to the lungs was also inhibited.

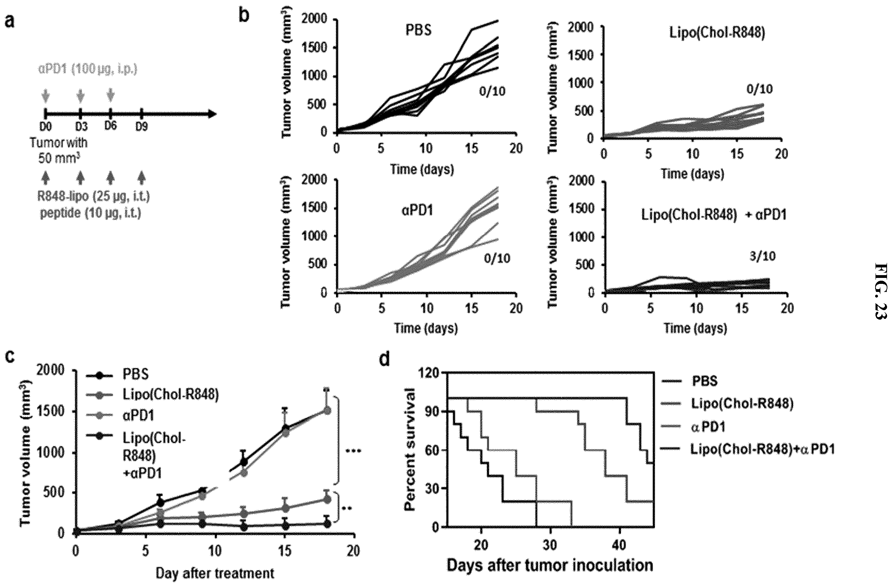

As shown in FIG. 23, in the case of the 4T1 animal model, it was also confirmed that tumor growth is inhibited and a survival rate is increased in the experimental group co-administered an immune checkpoint inhibitor.

### 6.3. Verification of anticancer effect of co-administration of cholesterol-toll-like receptor 7/8 agonist complex and chemotherapeutic agent

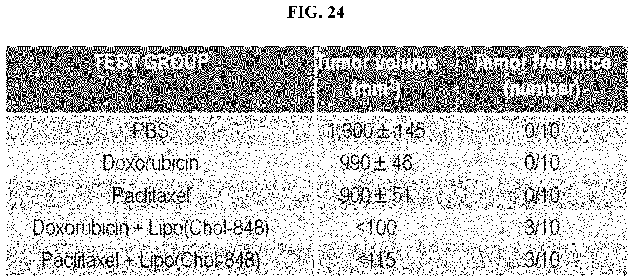

To confirm an anticancer effect in the co-administration of a cholesterol-toll-like receptor 7/8 agonist complex and a chemotherapeutic agent, a 4T1 animal model was prepared in the same method as in Example 6.2, and doxorubicin or paclitaxel as a chemotherapeutic agent and a nanoliposome including a cholesterol-resquimod complex were co-administered. In addition, after two weeks, a cancer size was measured. The result is shown in FIG. 24.

As shown in FIG. 24, compared to the group administered a chemotherapeutic agent alone, in the case of co-administration, it was confirmed that cancer growth was not only effectively inhibited, but the number of tumor-free mice also increased.

From the above results, since the toll-like receptor 7/8 agonist-cholesterol complex can be prevented from penetrating into blood, side effects of the conventional toll-like receptor 7/8 agonist can be considerably reduced, and cytotoxicity is not exhibited, it was confirmed that not only intracellular delivery efficacy does increases, but the degree of immune activity also increases due to effective separation from cholesterol compared to when a toll-like receptor 7/8 agonist is used alone. In addition, since it is based on cholesterol, the toll-like receptor 7/8 agonist-cholesterol complex may be easily prepared in the form of a nanoemulsion, nanoliposome or nanomicelle, and thus it is expected that the toll-like receptor 7/8 agonist may not only be widely used as an immune activation therapeutic agent, but also may increase an immune response of the antigen when used as an adjuvant in combination with an antigen. In addition, the toll-like receptor 7/8 agonist-cholesterol complex of the present invention induces immune activation of antigen-presenting cells (dendritic cells or macrophages), NK cells and T cells, and regulates the function of immune cells having an immunosuppressive action (regulatory T cells (Tregs), myeoloid-derived suppressor cells (MDSCs), or M2 macrophages) in a tumor microenvironment, and thus it is expected that they can be used as a composition for various types of anticancer treatment. In addition, specially, since an anticancer effect may considerably increase due to a synergistic effect in co-administration with materials for anticancer treatment such as an immune checkpoint inhibitor and a chemotherapeutic agent, the toll-like receptor 7/8 agonist-cholesterol complex may also be used in co-administration with various anticancer therapeutic agents.

It should be understood by those of ordinary skill in the art that the above description of the present invention is exemplary. Therefore, the exemplary embodiments described above should be interpreted as illustrative.

### [Industrial Applicability]

A toll-like receptor 7/8 agonist-cholesterol complex of the present invention is inactivated when administered into the body, but activated when separated from cholesterol in a tumor microenvironment and/or under a specific condition in cells. Therefore, it cannot only reduce a side effect such as a non-specific hypersensitive immune response, but can also be prepared in various formulations because it is complexed with cholesterol, and also inhibits absorption into a blood vessel, thereby inhibiting a side effect such as a cytokine storm. In addition, since the toll-like receptor 7/8 agonist-cholesterol complex alone can exhibit an anticancer effect by effectively regulating immune cells, but also considerably increase an effect of co-administration with various cancer therapeutic agents and immunotherapeutic agents, the toll-like receptor 7/8 agonist-cholesterol complex is expected to be effectively and widely applied to treatment of various diseases to which the toll-like receptor agonist can be applied.

## Claims

1. A conjugate of a toll-like receptor 7/8 agonist and cholesterol, in which cholesterol is linked to an amine group of an active site of a toll-like receptor 7/8 agonist, and the linkage is made in a separable form,
wherein the separable linkage is made by at least one selected from the group consisting of a carbamate, a disulfide, an ester, a peptide, and a combination thereof;
wherein the toll-like receptor 7/8 agonist includes at least one selected from the group consisting of an imidazoquinoline-based agonist, a hydroxyadenine-based agonist, a pteridone-based agonist, an aminopyrimidine-based agonist, a benzoazepine-based agonist, and a thia-oxoguanosine-based agonist.

2. The conjugate of claim 1, wherein the toll-like receptor 7/8 agonist is imiquimod, resiquimod, dactolisib, gardiquimod, sumanirole, motolimod, vesatolimod, loxoribine, SM360320, CL264 or 3M-003.

3. The conjugate of claim 1, wherein the linkage is cleaved in response to a tumor microenvironment, a temperature, endosomal and lysosomal enzymes, or pH in cells, and the active site of the toll-like receptor 7/8 agonist is exposed so that a kinetic function is recovered within 4 days.

4. A nanoparticle composition comprising the conjugate of any one of claim 1 to 3.

5. The nanoparticle composition of claim 4, wherein the nanoparticle includes at least one selected from the group consisting of a nanoliposome, a nanoemulsion, a nanomicelle, a solid nanoparticle, and a polymer nanoparticle.

6. An adjuvant composition comprising the conjugate of any one of claim 1 to 3 as an active ingredient.

7. A vaccine composition comprising the adjuvant composition of claim 6 and an antigen.

8. The vaccine composition of claim 7, wherein the antigen includes one or more selected from the group consisting of a protein, a recombinant protein, a glycoprotein, a gene, a peptide, a olysaccharide, a lipopolysaccharide, a polynucleotide, a cell, a cell lysate, a bacterium and a virus.

9. A composition for use in regulating an immune function, the composition comprising the conjugate of any one of claim 1 to 3 as an active ingredient.

10. The composition for use of claim 9, which activates at least one type of immune cells selected from the group consisting of antigen-presenting cells, natural killer cells (NK cells) and T cells.

11. The composition for use of claim 9, which regulates the function of one or more types of immune cells selected from the group consisting of regulatory T cells (Tregs), myeoloid-derived suppressor cells (MDSCs) and M2 macrophages.

12. A pharmaceutical composition for use in preventing or treating cancer, comprising the conjugate of any one of claim 1 to 3 as an active ingredient.

13. The pharmaceutical composition for use of claim 12, further comprising a chemotherapeutic agent or an immune checkpoint inhibitor.

14. The pharmaceutical composition for use of claim 12, which inhibits the proliferation, metastasis, recurrence of cancer, or resistance to anticancer therapy.

## Patentansprüche

1. Konjugat eines Toll-Like-Rezeptor-7/8-Agonisten und Cholesterin, in dem Cholesterin an eine Amingruppe einer aktiven Stelle eines Toll-Like-Rezeptor-7/8-Agonisten gebunden ist und die Bindung aus einer trennbaren Form hergestellt ist,
wobei die trennbare Bindung durch mindestens eines hergestellt ist, ausgewählt aus der Gruppe bestehend aus einem Carbamat, einem Disulfid, einem Ester, einem Peptid und einer Kombination daraus;
wobei der Toll-Like-Rezeptor-7/8-Agonist mindestens eines einschließt, ausgewählt aus der Gruppe bestehend aus einem Agonisten auf Imidazochinolin-Basis, einem Agonisten auf Hydroxyadenin-Basis, einem Agonisten auf Pteridon-Basis, einem Agonisten auf Aminopyrimidin-Basis, einem Agonisten auf Penzoazepin-Basis und einem Agonisten auf Thia-oxoguanosin-Basis.

2. Konjugat nach Anspruch 1, wobei der Toll-Like-Rezeptor-7/8-Agonist Limiquimod, Resiquimod, Dactolisib, Gardiquimod, Sumanirole, Motolimod, Vesatolimod, Loxoribine, SM360320, CL264 oder 3M-003 ist.

3. Konjugat nach Anspruch 1, wobei die Bindung zerklüftet ist als Reaktion auf eine Tumor-Mikrobewegung, eine Temperatur, endosomale und lysosomale Enzyme oder pH in Zellen und die aktive Stelle des Toll-Like-Rezeptor-7/8-Agonisten so exponiert ist, dass eine kinetische Funktion innerhalb von 4 Tagen wiederhergestellt ist.

4. Nanopartikelzusammensetzung, die das Konjugat nach einem von Anspruch 1 bis 3 umfasst.

5. Nanopartikelzusammensetzung nach Anspruch 4, wobei die Nanopartikel mindestens eines einschließen, ausgewählt aus der Gruppe bestehend aus Nanoliposom, einer Nanoemulsion, einer Nanomizelle, einem festen Nanopartikel und einem Polymer-Nanopartikel.

6. Adjuvans-Zusammensetzung, die das Konjugat nach einem von Anspruch 1 bis 3 als Wirkstoff umfasst.

7. Impfzusammensetzung, die die Adjuvans-Zusammensetzung nach Anspruch 6 und ein Antigen umfasst.

8. Impfzusammensetzung nach Anspruch 7, wobei das Antigen eines oder mehreres einschließt, ausgewählt aus der Gruppe bestehend aus einem Protein, einem rekombinanten Protein, einem Glykoprotein, einem Gen, einem Peptid, einem Oligosaccharid, einem Lipopolysaccharid, einem Polynukleotid, einer Zelle, einem Zelllysat, einem Bakterium und einem Virus.

9. Zusammensetzung zur Verwendung zur Regulierung einer Immunfunktion, wobei die Zusammensetzung das Konjugat nach einem von Anspruch 1 bis 3 als Wirkstoff umfasst.

10. Zusammensetzung zur Verwendung nach Anspruch 9, die mindestens einen Typ von Immunzellen aktiviert, ausgewählt aus der Gruppe bestehend aus antigenpräsentierenden Zellen, natürlichen Killerzellen (NK-Zellen) und T-Zellen.

11. Zusammensetzung zur Verwendung nach Anspruch 9, die die Funktion von einem oder mehreren Typen von Immunzellen reguliert, ausgewählt aus der Gruppe bestehend aus regulatorischen T-Zellen (Tregs), myeloid-abgeleiteten Suppressorzellen (MDSCs) und M2-Makrophagen.

12. Pharmazeutische Zusammensetzung zur Verwendung in der Prävention oder Behandlung von Krebs, die das Konjugat nach einem von Anspruch 1 bis 3 als Wirkstoff umfasst.

13. Pharmazeutische Zusammensetzung zur Verwendung nach Anspruch 12, weiter umfassend ein Chemotherapeutikum oder einen Immun-Checkpoint-Inhibitor.

14. Pharmazeutische Zusammensetzung zur Verwendung nach Anspruch 12, die die Proliferation, Metastase, Rezidiv von Krebs oder Resistenz gegen Krebstherapie unterbindet.

## Revendications

1. Conjugué d'un agoniste du récepteur de type Toll 7/8 et du cholestérol, dans lequel le cholestérol est lié à un groupe amine d'un site actif d'un agoniste du récepteur de type Toll 7/8, et la liaison est réalisée sous forme séparée,
dans lequel la liaison séparée est réalisée par au moins un sélectionné parmi le groupe consistant en un carbamate, un disulfure, un ester, un peptide et une combinaison de ceux-ci ;
dans lequel l'agoniste du récepteur de type Toll 7/8 inclut au moins un sélectionné parmi le groupe consistant en un agoniste à base d'imidazoquinoline, un agoniste à base d'hydroxyadénine, un agoniste à base de ptéridone, un agoniste à base d'aminopyrimidine, un agoniste à base de benzoazépine et un agoniste à base de thia-oxoguanosine.

2. Conjugué selon la revendication 1, dans lequel l'agoniste du récepteur de type Toll 7/8 est imiquimod, résiquimod, dactolisib, gardiquimod, sumanirol, motolimod, vésatolimod, loxoribine, SM360320, CL264 ou 3M-003.

3. Conjugué selon la revendication 1, dans lequel la liaison est clivée en réponse à un microenvironnement tumoral, à une température, à des enzymes endosomales et lysosomales ou au pH dans les cellules, et le site actif de l'agoniste du récepteur de type Toll 7/8 est exposé de sorte qu'une fonction cinétique est rétablie dans un délai de 4 jours.

4. Composition de nanoparticules comprenant le conjugué selon l'une quelconque des revendications 1 à 3.

5. Composition de nanoparticules selon la revendication 4, dans laquelle la nanoparticule inclut au moins un sélectionné parmi le groupe consistant en un nanoliposome, une nanoémulsion, une nanomicelle, une nanoparticule solide et une nanoparticule polymérique.

6. Composition adjuvante comprenant le conjugué selon l'une quelconque des revendications 1 à 3 en tant que principe actif.

7. Composition vaccinale comprenant la composition adjuvante selon la revendication 6 et un antigène.

8. Composition vaccinale selon la revendication 7, dans laquelle l'antigène inclut un ou plusieurs éléments sélectionnés parmi le groupe consistant en une protéine, une protéine recombinante, une glycoprotéine, un gène, un peptide, un polysaccharide, un lipopolysaccharide, un polynucléotide, une cellule, un lysat cellulaire, une bactérie et un virus.

9. Composition destinée à la régulation d'une fonction immunitaire, la composition comprenant le conjugué selon l'une quelconque des revendications 1 à 3 en tant que principe actif.

10. Composition à utiliser selon la revendication 9, qui active au moins un type de cellules immunitaires sélectionné parmi le groupe consistant en des cellules présentatrices d'antigène, des cellules tueuses naturelles (cellules NK) et des lymphocytes T.

11. Composition pour une utilisation selon la revendication 9, qui régule la fonction d'un ou plusieurs types de cellules immunitaires sélectionnés parmi le groupe consistant en des lymphocytes T régulateurs (Tregs), des cellules suppressives d'origine myéloïde (MDSC) et des macrophages de type M2.

12. Composition pharmaceutique pour une utilisation dans la prévention ou le traitement du cancer, comprenant le conjugué selon l'une quelconque des revendications 1 à 3 en tant que principe actif.

13. Composition pharmaceutique pour une utilisation selon la revendication 12, comprenant en outre un agent chimiothérapeutique ou un inhibiteur de point de contrôle immunitaire.

14. Composition pharmaceutique pour une utilisation selon la revendication 12, qui inhibe la prolifération, les métastases, la récurrence du cancer ou la résistance au traitement anticancéreux.
